# EUROPÄISCHE PATENTANMELDUNG

(11) **EP 2 712 609 A1**
(43) Veröffentlichungstag der Anmeldung: **02.04.2014**
(21) Anmeldenummer: 12186103.3
(22) Anmeldetag: 26.09.2012
(51) Int. Cl.: A61K 8/87, A61Q 5/06, A61Q 19/00, A61Q 19/10

(54) **Polyurethanharnstoff-Mischung für die Haut-, sowie Haarkosmetik**

(71) Anmelder: Bayer MaterialScience AG, 51373 Leverkusen (DE)
(72) Erfinder: Viala, Sophie Dr., 50935 Köln (DE); Dörr, Sebastian, 40593 Düsseldorf (DE)
(74) Vertreter: BIP Patents

(57) **Zusammenfassung**

Die vorliegende Erfindung betrifft die Verwendung eines speziellen Polyurethanharnstoffs zur Herstellung von haut- und haarkosmetischen Zusammensetzungen, die Verwendung dieser kosmetischen Zusammensetzungen in der Haut- und Haarkosmetik, die Verwendung von den speziellen Polyurethanharnstoff enthaltenden Zusammensetzungen zur Erzielung eines Shape-Memory-Effekts auf Körpergewebe, sowie Verfahren zur Reinigung, zur Pflege und/oder zum Schutz der Haut, bzw. zum Erzielen eines stylenden Effekts der Haare mittels Dauerwellen, eines Effekts zur Glättung der Haare (Curl Relaxer), eines Effekts zum Einbringen von Locken in die Haare (Styling Wrap Lotion), eines haarverformenden Effekts, eines färbenden, reinigenden, oder pflegenden Effekts der Haare, bzw. zum Erzielen eines Shape Memory-Effekts auf Körpergewebe durch Auftragen von Zusammensetzungen enthaltend den speziellen Polyurethanharnstoff auf Körpergewebe.

## Beschreibung

Die vorliegende Erfindung betrifft die Verwendung eines speziellen Polyurethanharnstoffs zur Herstellung von haut- und haarkosmetischen Zusammensetzungen, die Verwendung dieser kosmetischen Zusammensetzungen in der Haut- und Haarkosmetik, die Verwendung von den speziellen Polyurethanharnstoff enthaltenden Zusammensetzungen zur Erzielung eines Shape-Memory-Effekts auf Körpergewebe, sowie Verfahren zur Reinigung, zur Pflege und/oder zum Schutz der Haut, bzw. zum Erzielen eines stylenden Effekts der Haare mittels Dauerwellen, eines Effekts zur Glättung der Haare (Curl Relaxer), eines Effekts zum Einbringen von Locken in die Haare (Styling Wrap Lotion), eines haarverformenden Effekts, eines färbenden, reinigenden, oder pflegenden Effekts der Haare, bzw. zum Erzielen eines Shape Memory-Effekts auf Körpergewebe durch Auftragen von Zusammensetzungen enthaltend den speziellen Polyurethanharnstoff auf Körpergewebe.

Verbraucher wünschen sich bei der Anwendungen von zahlreichen kosmetischen Formulierungen naturgemäß einen langhaltenden Effekt, ohne Kompromiss in Bezug auf die sensorischen Eigenschaften. Insbesondere erwarten Verbraucher eine gute Beständigkeit gegenüber Wasser, Luftfeuchtigkeit, Tränen oder Schweiß.

Aufgabe der vorliegenden Anmeldung war, eine haut- und haarkosmetische Zusammensetzung bereitzustellen, welche eine hohe Beständigkeit, insbesondere Wasserfestigkeit und Feuchtigkeitbeständigkeit und gleichzeitig einen hohen Tragekomfort aufweisst. Insbesondere sollte eine dekorative kosmetische Zusammensetzung bereit gestellt werden, die zudem eine einfache Auswaschbarkeit aufweist. Ebenso sollte eine Mascara Zusammensetzung bereitgestellt werden, welche einen ausgezeichneten Curl Effekt aufweisst.

Die PCT/EP2012/054976 beschreibt die Verwendung von speziellen Poyurethanharnstoffen zur Verwendung im haarkosmetischen Bereich, im Besonderen im Bereich des Stylens durch Haarfestiger und Schaumfestiger. Weitere haarkosmetische Bereiche werden nicht genannt. Eine Verwendung in hautkosmetischen Bereichen ist in keinster Weise Thema der Anmeldung.

Überraschend wurde nun gefunden, dass haut- und haarkosmetische Zusammensetzungen, die auf spezielle Polyurethanharnstoffen basieren, die oben geforderten Eigenschaften aufweisen.

Gegenstände der vorliegenden Anmeldung sind daher die Verwendung mindestens eines Polyurethanharnstoffs zur Herstellung von hautkosmetischen Zusammensetzungen, sowie von haarkosmetischen Zusammensetzungen, wobei letztere ausgewählt sind aus der Gruppe bestehend aus Egalisierungsmittel für Dauerwellen, Curl Relaxer, Styling Wrap Lotion, Haarverformungsmittel, Haarfärbemittel, Haarkuren, Conditioner und Shampoo, dadurch gekennzeichnet, dass der Polyurethanharnstoff erhältlich ist durch Umsetzung
a) einer einzigen Polyisocyanat-Komponente,
b) wenigstens einer polymeren Polyolkomponente,
c) wenigstens einer hydrophilierenden Komponente und
d) einer einzigen aminofunktionellen Kettenverlängerer-Komponente,
wobei die Polyisocyanat-Komponente a) mehr als 75 mol% Isophorondiisocyanat und die aminofunktionelle Kettenverlängerer-Komponente c) mehr als 75 mol % Isophorondiamin umfasst.

Weitere Gegenstände sind die Verwendung von kosmetischen Zusammensetzungen, enthaltend einen oder mehrere der oben genannten Polyurethanhaarstoffe in der Haut-, bzw. Haarkosmetik, wobei letztere das Stylen durch Dauerwellen, Curl Relaxer, Styling Wrap Lotions, Haarverformungsmittel, sowie das Färben, Reinigen und Pflegen durch Haarfärbemittel, Haarkuren, Conditioner und Shampoo umfasst.

Überraschend wurde auch gefunden, dass sich die oben genannten Polyurethanharnstoffe bzw. kosmetische Zusammensetzungen enthaltend die oben genannten Polyurethanharnstoffe besonders eignen zur Erzielung eines Shape-Memory-Effekts auf Körpergewebe, wie Haut und Haar.

So wurde gefunden, dass nach Auftragen einer kosmetischen Formulierung mit den erfindungsgemäßen Polyurethanharnstoffen ein Shape Memory Effekt erzielt wird, ohne dass freie Filme aus erfindungsgemäßen Polyurethanharnstoffen eine derartigen Shape Memory Effekt aufweisen.

Ein Shape-Memory-Effekt basiert auf Formgedächtnispolymeren. Als Formgedächtnispolymere (FGP, Shape-Memory-Polymers (SMP)) werden im allgemeinen Polymere bezeichnet, die sich nach einer Umformung an ihre frühere äußere Form scheinbar "erinnern" können und insofern ein Formgedächtnis besitzen. Um die frühere Form abzurufen, muss das FGP einem Stimulus ausgesetzt werden. Dieser Stimulus kann beispielsweise in einer Wärmezufuhr bestehen, indem das betreffende FGP direkt oder indirekt erwärmt wird. Eine direkte Erwärmung des FGP kann von außen durch heiße Luft, durch IR Einstrahlung, beispielsweise durch Exposition mit Sonnenlicht oder den Luftstrom eines Heißlüfters.

Unter spezifischen Eigenschaften einer FGP Komponente werden beispielsweise dessen Schmelztemperatur, sowie dessen Glasübergangstemperatur (T.), Kristallisationstemperatur der Weichsegmente eines Blockcopolymeren und daraus resultierende Formgedächtniseigenschaften, wie das Maß oder der Grad der erreichten Rückstellung (recovery), seine Rückstellrate, seine Rückstellgeschwindigkeit und seine Rückstelltemperatur oder Übergangstemperatur (T t.) verstanden. Weitere Parameter der FGP-Komponente der Vorrichtung sind beispielsweise sein Elastizitätsmodul, seine Härte und seine Scherfestigkeit.

Diese Werte ergeben sich beispielsweise aus der chemischen Natur des Formgedächtnispolymers oder des Formgedächtnispolymer-Komposits und sind dem Fachmann bekannt.

Weiterer Gegenstand der vorliegenden Anmeldung ist die Verwendung einer Zusammensetzung enthaltend mindestens einen Polyurethanharnstoff, der erhältlich ist durch Umsetzung
a) einer einzigen Polyisocyanat-Komponente,
b) wenigstens einer polymeren Polyolkomponente,
c) wenigstens einer hydrophilierenden Komponente und
d) einer einzigen aminofunktionellen Kettenverlängerer-Komponente,
wobei die Polyisocyanat-Komponente a) mehr als 75 mol% Isophorondiisocyanat und die aminofunktionelle Kettenverlängerer-Komponente c) mehr als 75 mol % Isophorondiamin umfasst,
zur Erzielung eines Shape-Memory-Effekts auf Körpergewebe.

Gegenstand ist auch ein Verfahren zur Erzielung eines Shape-Memory-Effekts auf Körpergewebe, bei dem kosmetische Zusammensetzungen, wie oben beschrieben, auf Haut oder Haare aufgetragen werden.

Gegenstand ist weiterhin ein Verfahren zur kosmetischen Behandlung von Körpergewebe, wobei
- eine Zusammensetzung auf das Körpergewebe aufgebracht wird, die mindestens einen Polyurethanharnstoff umfasst,
   - der erhältlich ist durch Umsetzung
      a) einer einzigen Polyisocyanat-Komponente,
      b) wenigstens einer polymeren Polyolkomponente,
      c) wenigstens einer hydrophilierenden Komponente und
      d) einer einzigen aminofunktionellen Kettenverlängerer-Komponente,
      wobei die Polyisocyanat-Komponente a) mehr als 75 mol% Isophorondiisocyanat und die aminofunktionelle Kettenverlängerer-Komponente c) mehr als 75 mol % Isophorondiamin umfasst,
      und
   - der in der Lage ist, nach Aufbringung auf Körpergewebe und nach Durchführung der im Folgenden beschriebenen Behandlung dem Körpergewebe einen Formgedächtnis-Effekt zu verleihen,
- vorher, gleichzeitig oder nach dem Aufbringen der Zusammensetzung das Körpergewebe in eine bestimmte Form (permanente Gedächtnisform) gebracht wird und
wobei nach einer gewollten oder ungewollten Deformation der Gedächtnisform die ursprüngliche Gedächtnisform durch eine physikalische Stimulation im Wesentlichen wiederherstellbar ist.

Ein weiterer Gegenstand ist ein Verfahren wie zuvor beschrieben, wobei es sich bei dem Körpergewebe um Haut oder Haare handelt.

Gegenstände sind weiterhin Verfahren wie zuvor beschrieben, wobei die Zusammensetzungen als weitere Komponenten Öle, Fette , Wachse, Verdickungsmittel, Emulgatoren, UV-Filter, Farbstoffe, Konditionierungsmittel, Tenside, Filmbildner, Lösungsmittel, Treibgase, Wirkstoffe und oder sonstige Hilfs- und/oder Zusatzstoffe enthalten.

Erfindungsgemäß wird unter einer aminofunktionellen Kettenverlängerer-Komponente eine Komponente verstanden, die wenigstens eine Verbindung mit zwei isocyanatreaktiven Aminogruppen und keine hydrophilierenden Gruppen umfasst.

Gemäß einer ersten bevorzugten Ausführungsform des erfindungsgemäßen Polyurethanharnstoffs kann die Polyisocyanat-Komponente a) ≥ 80 mol%, bevorzugt ≥ 85 mol%, weiter bevorzugt 95 mol% und besonders bevorzugt 100 mol% IPDI umfassen.

Weitere - zusätzlich zu IPDI in einem molaren Anteil von weniger als 25 mol% einsetzbare - Polyisocyanate der Komponente a) sind die dem Fachmann an sich bekannten aromatischen, araliphatischen, aliphatischen oder cycloaliphatischen Polyisocyanate einer NCO-Funktionalität von ≥ 2.

Beispiele solcher Polyisocyanate sind 1,4-Butylendiisocyanat, 1,6-Hexamethylendiisocyanat (HDI), 2,2,4 und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis(isocyanatomethyl)benzol (XDI), AIkyl-2,6-diisocyanatohexanoat (Lysindiisocyanate) mit C1-C8-Alkylgruppen, sowie 4-Isocyanatomethyl-1,8-octandiisocyanat (Nonantriisocyanat) und Triphenylmethan-4,4',4''-triisocyanat.

Neben den vorstehend genannten Polyisocyanaten können anteilig auch modifizierte Diisocyanate oder Triisocyanate mit Uretdion-, Isocyanurat-, Urethan-, Allophanat-, Biuret-, Iminooxadiazindion- und/oder Oxadiazintrionstruktur mit eingesetzt werden.

Bevorzugt handelt es sich um Polyisocyanate oder Polyisocyanatgemische der vorstehend genannten Art mit ausschließlich aliphatisch und / oder cycloaliphatisch gebundenen Isocyanatgruppen und einer mittleren NCO-Funktionalität der Mischung von 2 bis 4, bevorzugt von 2 bis 2,6 und besonders bevorzugt von 2 bis 2,4.

Besonders bevorzugt werden - falls neben IPDI weitere Polyisoyanate in der Komponente a) eingesetzt werden 1,6-Hexamethylendiisocyanat, die isomeren Bis(4,4'-isocyanatocyclohexyl)methane, sowie deren Mischungen verwendet.

Bevorzugt ist ebenfalls, wenn die polymere Polyolkomponente b) ein zahlenmittlere Molekulargewichte von ≥ 400 und ≤ 8000 g/mol, besonders bevorzugt von 600 bis 3000 g/mol eingesetzt und /oder eine mittlere OH-Funktionalitäten von 1,5 bis 6,. bevorzugt von 1,8 bis 3 und besonders bevorzugt von 1,9 bis 2,1 aufweist.

Ebenfalls vorteilhaft ist, wenn die polymere Polyolkomponente b) einen Polyester, bevorzugt einen Polyester basierend auf Adipinsäure umfasst oder daraus besteht.

Mögliche Bestandteile der polymeren Polyolkomponente b) sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in b) einzeln oder in beliebigen Mischungen untereinander eingesetzt werden.

Polyesterpolyole sind beispielsweise die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele hierfür geeigneter Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Neopentylglykol und Hydroxypivalinsäureneopentylglykolester bevorzugt sind. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols größer als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure, ganz besonders bevorzugt ist Adipinsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

In der Komponente b) können auch Hydroxylgruppen aufweisende Polycarbonate, bevorzugt Polycarbonatdiole, mit zahlenmittleren Molekulargewichten Mn von 400 bis 8000 g/mol, bevorzugt von 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art. Die Hydroxylgruppen aufweisenden Polycarbonate sind bevorzugt linear gebaut.

Ebenfalls können in der Komponente b) Polyetherpolyole eingesetzt werden. Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxide und/oder Epichlorhydrin an di- oder polyfunktionelle Startermoleküle.

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol, 1,4-Butandiol, Diethylenglykol und Butyldiglykol.

Gemäß einer weiteren vorteilhaften Ausführungsform des erfindungsgemäßen Polyurethanharnstoffs ist vorgesehen, dass die hydrophilierende Komponente c) eine anionisch hydrophilierende Komponente und bevorzugt ein Sulfonat ist.

Geeignete anionisch bzw. potentiell anionisch hydrophilierenden Verbindungen der Komponente c) sind Verbindungen, die mindestens eine isocyanatreaktive Gruppe wie eine Hydroxylgruppe oder Amionogruppe aufweisen sowie mindestens eine Funktionalität wie z.B. -COO-M⁺, -SO₃-M⁺, - PO(O-M⁺)₂ mit M⁺ beispielsweise gleich Metallkation, H⁺, NH₄⁺, NHR₃⁺, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann. Geeignete anionisch oder potentiell anionisch hydrophilierende Verbindungen sind Mono- und Dihydroxycarbonsäuren, Mono- und Dihydroxysulfonsäuren, sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und NaHSO₃, wie es in DE-A 2 446 440, Seite 5 - 9, Formel I-III beschrieben ist. Bevorzugte anionische oder potentiell anionische Hydrophilierungsmittel der Komponente c) sind solche der vorstehend genannten Art, die über Carboxylat- bzw. Carbonsäuregruppen und/oder Sulfonatgruppen verfügen.

Geeignete nichtionisch hydrophilierende Verbindungen der Komponente c) sind z.B. Polyoxyalkylenether, die mindestens eine Hydroxy- oder Aminogruppe, bevorzugt mindestens eine Hydroxygruppe enthalten.

Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38 beschrieben). Diese Verbindungen sind entweder reine Polyethylenoxidether oder gemischte Polyalkylenoxidether, wobei sie dann aber mindestens 30 mol-%, bevorzugt mindestens 40 mol-%, bezogen auf alle enthaltenen Allcylenoxideinheiten an Ethylenoxideinheiten, enthalten. Besonders bevorzugte nichtionische Verbindungen sind monofunktionelle, gemischte Polyalkylenoxidpolyether, die 40 bis 100 mol-% Ethylenoxid- und 0 bis 60 mol-% Propylenoxideinheiten aufweisen.

Zur Hydrophilierung können auch Mischungen aus anionischen bzw. potentiell anionischen Hydrophilierungsmitteln und nichtionischen Hydrophilierungsmitteln verwendet werden.

In Weiterbildung der Erfindung ist vorgesehen, dass die aminofunktionelle Kettenverlängerer-Komponente d) ≥ 85 mol%, bevorzugt ≥ 95 mol% und besonders bevorzugt 100 mol% IPDA umfassen kann.

Als weitere Bestandteile der aminofunktionelle Kettenverlängerer d) können neben IPDI weitere NH₂- und / oder NH-funktionelle Verbindungen eingesetzt werden.

Bevorzugt wird die Kettenverlängerung/-terminierung vor der Dispergierung in Wasser durchgeführt, dabei reagieren die Isocyanatgruppen mit dem Kettenverlängerer zu Harnstoffgruppen.

Geeignete Komponenten - die zusätzlich zu IPDA in einem molaren Anteil von weniger als 25% eingesetzt werden können - sind Di- oder Polyamine wie 1,2-Ethylendiamin, 1,2- und 1,3-Diaminopropan, 1,4-Diaminobutan, 1,6-Diaminohexan, 2,2,4- und 2,4,4-Trimethylhexamethylendiamin, 2-Methylpentamethylendiamin, Diethylentriamin, Triaminononan, 1,3- und 1,4-Xylylendiamin, α,α,α',α'-Tetramethyl-1,3- und -1,4-xylylendiamin und 4,4-Diaminodicyclohexylmethan und/oder Dimethylethylendiamin. Ebenfalls möglich sind Hydrazin oder sowie Hydraziden wie Adipinsäuredihydrazid.

Zur Herstellung des erfindungsgemäßen Polyurethanharnstoffs können außer den Komponten a) bis d) auch weitere Bausteine eingesetzt werden.

Beispiele sind hydroxyfunktionellen Verbindungen mit Molekulargewichten von 62 bis 399 g/mol wie beispielsweise Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit. Weiterhin können monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin.

Bevorzugt werden außer den Komponenten a) bis d) keine weiteren Bausteine zur Herstellung des erfindungsgemäßen Polyurethanharnstoffs eingesetzt.

Die Herstellung des erfindungsgemäßen Polyurethanharnstoffs kann nach den dem Fachmann bekannten Verfahren in einer oder mehreren Stufe/-n in homogener oder bei mehrstufiger Umsetzung erfolgen und teilweise in disperser Phase durchgeführt werden. Bevorzugt erfolgt nach vollständig oder teilweise durchgeführter Polyaddition aus a) bis d) ein Dispergier-, Emulgier- oder Lösungsschritt. Im Anschluss erfolgt gegebenenfalls eine weitere Polyaddition oder Modifikation in disperser oder gelöster (homogener) Phase. Dabei können alle aus dem Stand der Technik bekannten Verfahren wie z. B. Prepolymer-Mischverfahren, Acetonverfahren oder Schmelzdispergierverfahren verwendet werden. Bevorzugt wird nach dem Aceton-Verfahren gearbeitet.

Die erfindungsgemäßen haut- und haarkosmetischen Zusammensetzungen, enthaltend den erfindungsgemäßen Polyurethanharnstoff, liegen als wässrige oder wässrige-alkoholische Löungen, Öl-in-Wasser-, Silikon-in-Wasser, Wasser-in-Öl oder Wasser-in-Silkonemulsion und Mischform, Multiple Emulsion wie zum Beispiel Öl-in-Wasser-in-Öl-, Wasser-in-Öl-in-Wasser- Emulsion, Polymer stabilisierte Emulsion (so genannte Hydrodispersion), feststoffstabilisierte Emulsion (auch PickeringEmulsion genannt), PIT-Formulierung und Pulver in Form von Cremen, Lotionen, Schäumen, Sprays (Pump-Spray oder Aerosol), Gelen, Gelsprays, Ölen, Ölgelen, Mousse, "loose Pulver" , Kompaktpulver oder Stiftformulierungen für Anwendung auf Haar und/oder Haut vor.

Die erfindungsgemäßen kosmetischen Zusammensetzungen enthalten den erfindungsgemäßen Polyurethanharnstoff sowie gegebenenfalls in der Kosmetik übliche Wirkstoffe und Hilfsmittel, die aus der Gruppe, bestehend aus Emulgatoren; Tensiden; Konservierungsmitteln; Parfümölen; kosmetischen Wirkstoffen, wie Phytantriol, Vitamin A, E und C, Retinol, Bisabolol, Panthenol; organischen oder inorganischen Lichtschutzmitteln; Bleichmitteln, Färbemitteln; Tönungsmitteln; Bräunungsmitteln; Stabilisatoren; pH-Wert-Regulatoren; Farbstoffen; Salzen; Verdickern; Gelbildnern; Konsistenzgebem; Silikonen; Feuchthaltemitteln; wie Glycerin, Konditioniermitteln; Filmbildnern; Rückfettem und weiteren üblichen Additiven, ausgewählt werden.

Hautkosmetische Zusammensetzungen:

Im Sinne der Erfindung kann die kosmetische Zusammensetzung eine hautkosmetische Zusammensetzung sein. Eine hautkosmetische Zusammensetzung ist definiert als ein kosmetisches Mittel zur Reinigung, Pflege und zum Schutz der Haut. Im Sinne der vorliegenden Erfindung sind hautkosmetische Zusammensetzungen Hautpflegeprodukt, Sonnenschutzmittel, After-sun-Präparate, Selbstbräuner, dekorative Kosmetik, Wasch-, Dusch- und Badepräparate zur Anwendung auf der Haut, Gesichtwasser, Gesichtmasken, Insekten-Repellent-Präparate, Fußpflegemittel, Rasiermittel, Haarentfernungsmittel, Intimpflegemittel, Babypflegemittel, Deodorantien und Antitranspirantien.

Bevorzugte hautkosmetische Zusammensetzung im Sinne der vorliegenden Erfindung sind Hautpflegeprodukte, Sonnenschutzmittel, Selbstbräunungsmittel, dekorative Kosmetik, Insekten-Repellent-Präparate und Deodorantien und Antitranspirantien.

Weitere Gegenstände der Anmeldung sind Verfahren zur Reinigung, zur Pflege und/oder zum Schutz der Haut durch Auftragen einer Zusammensetzung enthaltend mindestens einen Polyurethanharnstoff, der/die erhältlich ist/sind wie oben beschrieben auf die Haut. Die Zusammensetzung kann zumindest teilweise auf dem Körpergewebe verbleiben.

Bei einem Hautpflegeprodukt handelt es ich um eine kosmetische Zusammensetzung zum Auftragen auf die Haut, das Gesicht oder/und den Körper zum Schutz gegen Hautveränderungen, beispielweise Hautalterung, Austrocknung usw.

Entsprechend ihrem Aufbau können die erfindungsgemäßen Zusammensetzungen beispielweise als Gesichtscreme, Tages- oder Nachtcreme, Augencreme, Antifaltencreme, Whitening-Produkte, Körperlotion, Tränkungsmedium, After-sun-Präparate usw. verwendet werden. Es ist gegebenenfalls möglich, dass die erfindungsgemäßen Zusammensetzungen als pharmazeutisches Produkt verwendet werden.

Insekten-Repellent-Präparate sind im Rahmen der vorliegenden Erfindung Präparate, die zur Abwehr und Vertreibung von Insekten, insbesondere von Mücken, Zecken und Milben, äußerlich verwendet werden. In solchen Formulierungen werden Wirkstoffe eingesetzt, welche die Insekten aufgrund der Bildung eines Duftmantels über der Haut von der Haut entfernt halten.

Bei einem Sonnenschutzmittel handelt es sich um eine Zusammensetzung zum Schutz der Haut gegenüber kurzwelliger und längerwelliger Sonnenstrahlung. Ein Sonnenschutzmittel enthält mindestens eine Lichtschutzfiltersubtanz (UVA-, UVB- und/oder Breitbandfilter).

Die erfindungsgemäßen Zusammensetzungen können Hautpflegeprodukte sein, enthaltend einen erfindungsgemäßen Polyurethanharnstoff, Feuchthaltmittel und gegebenenfalls weitere kosmetische und/oder dermatologische Wirk-, Hilfs- und Zusatzstoffe.

Die erfindungsgemäßen Zusammensetzungen können als Insekten-Reppelent-Präparate, enthaltend mindestens einen erfindungsgemäßen Polyurethanharnstoff, Insect-Repellent-Wirkstoffe und gegebenenfalls weitere kosmetische und/oder dermatologische Wirk-, Hilfs- und Zusatzstoffe, ausgebildet sein.

Vorteilhaft werden als Insect-Repellent-Wirkstoffe 3-(N-n-Butyl-N-acetyl-amino)-propionsäureethylester (unter der Handelsbezeichnung Repellent 3535 erhältlich), N,N-Ditethyl-m-toluamid (so genannt DEET) und 2-(2-Hydroxyethyl)-piperidin-1-carbonsäure-2-butylester (unter der Handelsbezeichnung Bayrepel^{®} erhältlich) eingesetzt.

Die erfindungsgemäßen Zusammensetzungen können auch als Sonnenschutzmittel sein, enthaltend mindestens einen erfindungsgemäßen Polyurethanharnstoff, mindestens eine oder mehrere Lichtschutzfiltersubtanzen und gegebenenfalls weitere kosmetische und/oder dermatologische Wirk-, Hilfs- und Zusatzstoffe, ausgebildet sein.

Lichtschutzfiltersubtanzen können aus der Gruppe, bestehend aus den UVA-, UVB-, Breitbandfiltern und deren Mischungen, ausgewählt werden.

Die erfindungsgemäßen Zusammensetzungen können als Selbstbräunungsmittel sein, enthaltend mindestens einen erfindungsgemäßen Polyurethanharnstoff, mindestens ein oder mehrere selbtsbräunende Subtanzen und gegebenenfalls weitere kosmetische und/oder dermatologische Wirk-, Hilfs- und Zusatzstoffe, ausgebildet sein.

Selbstbräunungsmittel enthalten mindestens ein oder mehrere selbtsbräunende Subtanzen, die bevorzugt ausgewählt werden aus der Gruppe, bestehend aus Glycerolaldehyd, Hydroxymethylglyoxal, γ-Dialdehyd, Erythrulose, 5-Hydroxy-1,4-naphtochionon, 2-Hydroxy-1,4-naphtochinon, 1-, 3-Dihydorxyaceton (DHA), 6-Aldo-D-Fructose und Ninhydrin.

Bei einer dekorativen kosmetischen Formulierung handelt es sich um eine kosmetische Zusammensetzung zur farblichen Gestaltung der menschlichen Haut, Schleimhaut, Semi-Schleimhaut, des Haar und des Nagels. Die erfindungsgemäße dekorative Formulierung kann ein Gesichts-Make-up (Foundation), eine getönte (Tages-)Creme, ein Blush, ein Rouge, eine Wimperntusche, ein Eyeliner, ein Kajal, ein Lidschatten, ein Lippenstift, ein Lip-Gloss zur farblichen Veränderung oder zum Schminken des Körpers gegen Augenringe, inhomogenen Teint oder weitere Unvollkommenheiten der Haut wie Rötungen, Flecken, Falten oder Pickel sein. Die Liste von dekorativen Produkten ist im Rahmen der vorliegenden Erfindung selbstverständlich nicht limitierend zu verstehen.

Die erfindungsgemäßen Zusammensetzungen können als dekorative kosmetische Zusammensetzungen sein, enthaltend mindestens einen erfindungsgemäßen Polyurethanharnstoff, mindestens einem Farbstoff und gegebenenfalls weitere kosmetische und/oder dermatologische Wirk-, Hilfs- und Zusatzstoffe, ausgebildet sein. Die Farbstoffe können ausgewählt werden aus der Gruppe, bestehend aus löslichen Farbstoffen; anorganischen Pigmenten, wie zum Beispiel Eisenoxiden und Chromoxiden; Ultramarin; Manganviolett; organischen Pigmenten und Perlmutt.

Die erfindungsgemäßen Zusammensetzungen können als Deodorantien und Antitranspirantien, enthaltend mindestens einen erfindungsgemäßen Polyurethanharnstoff, desodorierend wirkende Substanzen und/oder Antitranspirantwirkstoffe und gegebenenfalls weitere kosmetische und/oder dermatologische Wirk-, Hilfs- und Zusatzstoffe, ausgebildet sein.

Die erfindungsgemäßen hautkosmetischen Zusammensetzungen können fest (Stick), flüssig (Lotion, Pflegeöl) oder halbfest (Creme, Salben oder gelartige Produkte) sein. Die Zusammensetzungen können beispielweise in Form einer Öl-in-Wasser-, Silikon-in-Wasser- Wasser-in-Öl-, Wasser-in-Silikon-, Öl-in-Wasser-in-Öl- oder Wasser-in-Öl-in-Wasser-Emulsion vorliegen. Die Zusammensetzung können ferner mit einem Treibgas aufgeschäumt werden (so genanntes Mousse). Die oben erwähnten Emulsionen können durch O/W-, W/O- oder W/Si-Emulgator, Verdicker (wie beispielweise im Fall einer Hydrodispersion) oder Feststoffe (wie beispielweise Pickeringemulsion) stabilisiert sein. Die erfindungsgemäße Formulierung kann in Form von "loose Pulver" oder Kompaktpulver vorliegen.

### Haarkosmetische Zusammensetzung:

Im Sinne der Erfindung kann die kosmetische Zusammensetzung eine haarkosmetische Zusammensetzung sein, ausgewählt aus der Gruppe, bestehend aus Egalisierungsmittel für Dauerwellen, Curl Relaxer, Styling Wrap Lotion, Haarverformungsmittel, Haarfärbemittel, Haarkuren, Conditioner und Shampoo.

Bezüglich der Erzeugung des Shape-Memory-Effekts kann die haarkosmetische Zusammensetzung auch ein Haarfestiger sein.

Weitere Gegenstände der Anmeldung sind Verfahren zum Erzielen eines stylenden Effekts der Haare mittels Dauerwellen, eines Effekts zur Glättung der Haare (Curl Relaxer), eines Effekts zum Einbringen von Locken in die Haare (Styling Wrap Lotion), eines haarverformenden Effekts, eines färbenden, reinigenden, oder pflegenden Effekts der Haare durch Auftragen einer Zusammensetzung enthaltend mindestens einen Polyurethanharnstoff, der/die erhältlich ist/sind wie oben beschrieben auf die Haare. Die Zusammensetzung kann zumindest teilweise auf dem Körpergewebe verbleiben.

Die erfindungsgemäßen haarkosmetischen Zusammensetzungen enthalten mindestens einen erfindungsgemäßen Polyurethanharnstoff, mindestens eine Komponente, die ausgewählt ist aus der Gruppe, bestehend aus Konditioniermitteln, Filmbildnern und Tensiden, und gegebenenfalls weiteren kosmetischen und/oder dermatologischen Wirk-, Hilfs- und Zusatzstoffen.

Mögliche Inhaltstoffe der erfindungsgemäßen haut- und haarkosmetischen Zusammensetzungen:
Öle, Fette, Wachse:

Die erfindungsgemäßen haut- und haarkosmetischen Zusammensetzungen enthalten bevorzugt weitere nicht flüchtige und/oder flüchtige Öle, Fette und/oder Wachse.

Nicht flüchtige Öle und Fette werden vorteilhaft ausgewählt aus der Gruppe, bestehend aus Ölen und Fetten aus mineralischer, tierischer, pflanzlicher oder synthetischer Herkunft; polaren oder unpolaren Ölen und deren Gemischen. Nicht flüchtige Öle und Fette der erfindungsgemäßen Zusammensetzungen können vorteilhaft ausgewählt werden aus folgender Substanzgruppe:
Mineralöle, polare Öle, wie Triglyceride der Caprin- oder der Caprylsäure, ferner natürliche Öle wie z.B. Rizinusöl;
Fette, natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
Alkylbenzoate; Silikonöle wie Dimethylpolysiloxane, Diethylpolysiloxane, Diphenylpolysiloxane sowie Mischformen daraus.

Die polaren Öle werden vorteilhaft ausgewählt aus der Gruppe, bestehend aus:
a) Estern aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen.
b) Estern aus aromatischen Carbonsäuren und gesättigten und/oder ungesättigten, verzweigten und/oder unverzweigten Alkoholen einer Kettenlänge von 3 bis 30 C-Atomen.
   Solche Esteröle können dann vorteilhaft ausgewählt werden aus der Gruppe, bestehend aus:
   Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, Isotridecylisononan o at, 2-Ethylhexylpalmitat, 2-Ethylhexyllaurat, 2-Ethylhexylisostearat, 2-Hexyldecylstearat, 2-Octyldodecylpalmitat, 2-Ethylhexylcocoat, Oleyloleat, Oleylerucat, Erucyloleat, Erucylerucat Dicaprypyp Carbonat (Cetiol CC) und Cocoglyceride (Myritol 331) sowie synthetische, halbsynthetische und natürliche Gemische solcher Ester, z.B. Jojobaöl;
c) Alkylbenzoate (C12-15-Alkylbenzoate (Finsolv^{®} TN von Finetex)) oder 2-Phenylethylbenzoate (X-Tend 226 von Ashland).
d) Lecithine und den Fettsäuretriglyceride, insbesondere Triglycerinester gesättigter und/oder ungesättigter, verzweigter und/oder unverzweigter Alkancarbonsäuren einer Kettenlänge von 8 bis 24, insbesondere 12 bis 18 C-Atomen.
   Beispielweise können die Fettsäuretriglyceride ausgewählt werden aus der Gruppe, bestehend aus Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl, Aprikosenkernöl, Avocadoöl und dergleichen.
e) Dialkylether und Dialkylcarbonate, wobei z.B. Dicaprylylether (Cetiol^{®} OE der Firma BASF) und/oder Dicaprylylcarbonat (beispielsweise Cetiol^{®} CC der Firma BASF) bevorzugt sind.
f) gesättigte oder ungesättigte, verzweigte oder unverzweigte Alkohole, wie zum Beispiel Octyldodecanol.

Nicht flüchtige Öle können ebenfalls vorteilhaft auch unpolare Öle sein, welche ausgewählt werden aus der Gruppe, bestehend aus den verzweigten und unverzweigten Kohlenwasserstoffen, insbesondere Mineralöl, Vaselineöl, Paraffinöl, Squalan und Squalen; Polyolefinen, beispielweise Polydecene, hydrogenierte Polyisobutene, C13-16 Isoparaffin und Isohexadecan.

Unpolare nicht flüchtige Öle können aus den nicht flüchtigen Silikonölen ausgewählt werden.

Von den nicht flüchtigen Silikonölen können die Polydimethylsiloxane (PDMS), die gegebenenfalls phenyliert sind, wie Phenyltrimethicon, oder gegebenenfalls substituiert sind mit aliphatischen und/oder aromatischen Gruppen oder mit funktionellen Gruppen, beispielsweise Hydroxygruppen, Thiolgruppen und/oder Aminogruppen; mit Fettsäuren, Fettalkoholen oder Polyoxyalkylenen modifizierte Polysiloxane und deren Gemische angegeben werden.

Besondere vorteilhafte Öle sind 2-Ethylhexylisostearat, Octyldodecanol, Isotridecylisononanoat, Isoeicosan, 2-Ethylhexylcocoat, C12-15 Alkylbenzoat, Caprylic/Capric Triglycerid, Dicaprylylether, Mineral Öl, Dicaprylylcarbonate, Cocoglyceride, Butylenglykol Dicarprylate/Dicaprate, Hydrogenated Polyisobutene, Cetaryl Isononanoate, Isodecyl Neopentanoate, Squalan und C13-16 Isoparaffin.

Die erfindungsgemäßen Zusammensetzungen können ferner ein Wachs enthalten.

Im Sinne der vorliegenden Schrift ist ein Wachs als lipophile Fettsubstanz definiert, die bei Raumtemperatur (25°C) fest ist und bei einer Schmelztemperatur zwischen 30°C und 200°C eine reversible Zustandsänderung fest/flüssig zeigt. Oberhalb des Schmelzpunktes wird das Wachs niedrigviskos und mischbar mit Ölen.

Das Wachs wird vorteilhaft gewählt aus der Gruppen von natürlichen Wachsen wie beispielweise Baumwollwachs, Carnaubawachs, Candelillawachs, Espartoawachs, Japanwachs, Montanwachs, Zuckerrohrwachs, Bienenwachs, Wollwachs, Sumac, Reiskleiewachs, Schellack, Laurelwachs, Mikrowachse, Ceresin, Ozokerit, Ouricuriwachs, Korkfaserwachs, Lignitwachse, Berrenwachs, Sheabutter, Hydrogenated Jojobawachs oder synthetische Wachse wie Paraffinwachse, Polyethylenwachse, durch Fischer-Tropsch-Synthese hergestellte Wachse, hydrierte Öle, Fettsäureester und Glyceride, die bei 25 °C fest sind, Silikonwachse wie bis-PEG-12 Dimethicone Candelillate und Derivaten (Alkylderivate, Alkoxyderivate und/oder Ester von Polymethylsiloxan) und deren Gemischen. Die Wachse können in Form von stabilen Dispersionen von kolloidalen Wachspartikeln vorliegen, die nach bekannten Verfahren hergestellt werden können, beispielsweise gemäß "Microemulsions Theory and Practice", L.M. Prince Ed., Academic Press (1977), Seiten 21-32.

Die erfindungsgemäßen Zusammensetzungen können ferner ein flüchtiges Öl enthalten, das aus der Gruppe von flüchtigen Kohlenwasserstoffölen, silikonierten Ölen oder fluorierten Ölen ausgewählt wird.

Im Sinne der vorliegenden Erfindung ist ein flüchtiges Öl ein Öl, das im Kontakt mit der Haut bei Raumtemperatur und Atmosphärendruck in weniger als einer Stunde verdampft. Das flüchtige Öl ist bei Raumtemperatur flüssig und weist bei Raumtemperatur und Atmosphärendruck einen Dampfdruck von vorzugsweise 0,13 bis 40 000 Pa (10⁻³ bis 300 mg Hg), insbesondere 1,3 bis 13 000 Pa (0,01 bis 100 mm Hg), besonders bevorzugt 1,3 bis 13 00 Pa (0,01 bis 10 mm Hg), und einen Siedepunkt von vorzugsweise von 150 bis 260 °C, besonders bevorzugt 170 bis 250 °C, auf.

Unter einem Kohlenwasserstofföl wird ein Öl verstanden, das im Wesentlichen aus Kohlenstoffatomen und Wasserstoffatomen und gegebenenfalls Sauerstoffatomen oder Stickstoffatomen gebildet wird und keine Siliziumatome oder Fluoratome enthält, wobei es auch aus Kohlenstoffatomen und Wasserstoffatomen bestehen kann; es kann mindestens eine Estergruppe, Ethergruppe, Aminogruppe und/oder Amidgruppe enthalten.

Unter einem silikonierten Öl wird ein Öl verstanden, das mindestens ein Siliziumatom und insbesondere Si-O-Gruppen enthält.

Unter einem fluorierten Öl ist ein Öl zu verstehen, das mindestens ein Fluoratom enthält.

Das flüchtige Kohlenwasserstofföl kann unter den Kohlenwasserstoffölen mit einem Flammpunkt von im Allgemeinen 40 bis 102 °C, vorzugsweise 40 bis 55 °C, besonders bevorzugt 40 bis 50 °C, ausgewählt werden.

Beispielweise sind die flüchtigen Kohlenwasserstoffölen flüchtige Kohlenwasserstofföle mit 8 bis 16 Kohlenstoffatomen und deren Gemische, insbesondere verzweigte C₈₋₁₆-Alkane, wie die Isoalkane (die auch als Isoparaffine bezeichnet werden) mit 8 bis 16 Kohlenstoffatomen, insbesondere Isododecan, Isodecan und Isohexadecan, sowie beispielsweise die Öle, die unter den Handelsnamen Isopars^{®} oder Permetyls^{®} angeboten werden; und die verzweigten C₈₋₁₆-Ester, wie Isohexylneopentanoat und deren Gemische.

Besonders vorteilhaft sind die flüchtigen Kohlenwasserstofföle wie Isododecan, Isodecan und Isohexadecan.

Das flüchtige silikonierte Öl kann unter den silikonierten Ölen mit einem Flammpunkt von im Allgemeinen 40 bis 102 °C, vorzugsweise einem Flammpunkt über 55 °C und höchstens 95 °C, besonders bevorzugt im Bereich von 65 bis 95 °C, ausgewählt werden.

Beispielweise können für die flüchtigen silikonierten Öle die geradkettigen oder cyclischen Silikonöle mit 2 bis 7 Siliziumatomen genannt werden, wobei diese Silikone gegebenenfalls Alkyl- oder Alkoxygruppen mit 1 bis 10 Kohlenstoffatomen enthalten.

Besonders vorteilhaft sind die flüchtigen silikonierten Öle wie Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, Octamethyltrisiloxan, Decamethyltetrasiloxan, Dodecamethylpentasiloxan und deren Gemische.

Das flüchtige fluorierte Öl besitzt im Allgemeinen keinen Flammpunkt.

Beispielweise sind die flüchtigen fluorierten Öle Nonafluorethoxybutan, Nonafluormethoxybutan, Decafluorpentan, Tetradecafluorhexan, Dodecafluorpentan und Mischungen davon.

Falls vorhanden kann die Fettphase der erfindungsgemäßen Zusammensetzungen ein nicht flüchtiges Öl und/oder flüchtiges Öl, Fette und Wachse enthalten. Die O/W- Zusammensetzung enthält vorzugsweise 0,01 bis 45 Gew.-% Öle, besonders bevorzugt 0,01 bis 20 Gew-% Öle, jeweils bezogen auf das Gesamtgewicht der Zusammensetzung. Die W/O- oder W/Si-Zusammensetzung enthält vorzugsweise mindestens 20 Gew.-% Öle, bezogen auf das Gesamtgewicht der Zusammensetzung.

### Verdickungsmittel:

Die erfindungsgemäßen Zusammensetzungen können, sofern eine wässrige Phase enthalten ist, vorteilhaft Verdicker (der Wasserphase) enthalten. Vorteilhafte Verdicker sind:
- Homo- oder Copolymere von Vernetzter oder nichtvernetzter Acrylsäure oder Methacrylsäure. Hierzu gehören vernetzte Hompolymere von Methacrylsäure oder Acrylsäure, Copolymerisate aus Acrylsäure und/oder Methacrylsäure und Monomeren, die von anderen Acryl- oder Vinylmonomeren abgeleitet sind, wie C10-30 Alkylacrylate, C1-30-Alkyhnethacrylate, Vinylacetat und Vinylpyrrolidone;
- Verdickende Polymere natürlicher Herkunft beispielweise auf Basis von Cellulose, Guargummi, Xanthan, Scleroglucan, Gellangummi, Rhamsan und Karayagummi, Alginate, Maltodextrin, Stärke und ihre Derivate, Johannisbrotkernmehl, Hyaluronsäure, Carrageenan;
- Nichtionische, anionische, kationische oder amphotere assoziative Polymere z.B. auf Basis von Polyethylenglycole und ihre Derivaten, oder Polyurethanen; und
- Vernetzte oder nichtvernetzte Homopolymere oder Copolymere auf Basis von Acrylamid oder Methacrylamid, wie Homopolymere von 2-Acrylamido-2-methylpropansulfonsäure, Copolymere von Acrylamid oder Methacrylamid und Methacryloyloxyethyltrimethylammoniumchlorid oder Copolymere von Acrylamid und 2-Acrylamido-2-methylpropansulfonsäure.

Besondere vorteilhafte Verdicker sind verdickende Polymere natürlicher Herkunft, Homo- oder Copolymere von vernetzter Acrylsäure oder Methacrylsäurer und vernetzte Copolymere von 2-Acrylamido-2-methylpropansulfonsäure.

Ganz besondere vorteilhafte Verdicker sind Xanthangummi, wie die unter den Bezeichnungen Keltrol^{®} und Kelza^{®} von der Firma CP Kelco angebotenen Produkte oder die Produkte der Firma RHODIA mit der Bezeichnung Rhodopol^{®}, und Guargummi, wie die unter der Bezeichnung Jaguar^{®} HP105 von der Firma RHODIA erhältlichen Produkte.

Ganz besondere vorteilhafte Verdicker sind vernetzte Homopolymere von Methacrylsäure oder Acrylsäure, die von der Firma Noveon unter den Bezeichnungen Carbopol^{®} 940, Carbopol^{®} 941, Carbopol^{®} 980, Carbopol^{®} 981, Carbopol^{®} ETD 2001, Carbopol^{®} EDT 2050, Carbopol^{®} 2984, Carbopol^{®} 5984 und Carbopol^{®} Ultrez 10; und von der Firma 3V unter den Bezeichnungen Synthalen^{®} K, Synthalen^{®} L und Synthalen^{®} MS im Handel erhältlich sind.

Ganz besondere vorteilhafte Verdicker sind vernetzte Copolymer von Acrylsäure oder Methacrylsäure und einem C₁₀₋₃₀-Alkylacrylat oder C₁₀₋₃₀-Alkylmethacrylat und Coplymere von Acrylsäure oder Methacrylsäure und Vinylpyrrolidone. Solche Copolymere sind beispielsweise von der Firma Lubrizol unter den Bezeichnungen Carbopol^{®} 1342, Carbopol^{®} 1382, Pemulen^{®} TR1 oder Pemulen^{®} TR2 und von der Firma Ashland unter den Bezeichnungen Ultrathix^{®} P-100 (INCI: Acrylic Acid/VP Crosspolymer) im Handel erhältlich.

Ganz besondere vorteilhafte Verdicker sind vernetzte Copolymere von 2-Acrylamido-2-methylpropansulfonsäure. Solche Copolymere sind beispielsweise von der Firma Clariant unter den Bezeichnungen Aristoflex^{®} AVC (INCI: Ammonium Acryloyldimethyltaurate/VP Copolymer) erhältlich.

Diese Verdicker sind im Allgemeinen in einer Konzentration von etwa 0 % bis 2 Gew.-%, vorzugsweise 0 % bis 1 Gew.-%, jeweils bezogen auf das Gesamtgewicht der Zusammensetzung, vorhanden.

Zur Stabilisierung der erfindungsgemäßen W/O Emulsionen gegen Sedimentierung oder Flockung der Wassertröpfchen kann ein Ölverdicker eingesetzt werden. Ölverdicker können auch als Konsistenzgeber in Öle-haltigen Zusammensetzungen eingesetzt werden.

Besonders vorteilhaft Ölverdicker sind organomodifizierte Tonen wie organomodifizierte Bentonite (Bentone^{®} 34 der Firma Rheox) organomodifizierte Hectorite (Bentone^{®} 27 und Bentone^{®} 38 der Firma Rheox) oder organomodifizierte Montmorillonit, hydrophobe pyrogene Kiesselsäure, wobei die Silanolgruppen mit Trimethylsiloxygruppen subtituiert sind (AEROSIL^{®} R812 der Firma Degussa) oder mit Dimethylsiloxygruppen oder Polydimethylsiloxan (AEROSIL^{®} R972, AEROSIL^{®} R974 von Degussa, CAB-O-SIL^{®} TS-610, "CAB-O-SIL^{®} TS-720 von Cabot), Magnesium- oder Aluminiumstearat, oder Styrol-Copolymere wie zum Beispiel Styrol-Butadiene-Styrol, Styrol-Isopropen-Styrol, Styrol-Ethylen/Buten-Styrol oder Styrol-Ethylen/Propen-Styrol.

Das Verdickungsmittel für die Fettphase kann in einer Menge von im Allgemeinen 0,1 bis 5 Gew. -%, vorzugsweise 0,4 bis 3 Gew.-%, jeweils bezogen auf das Gesamtgewicht der Zusammensetzung enthalten sein.

### Emulgatoren:

Die erfindungsgemäßen Zusammensetzungen in Form einer Emulsionen wie beispielweise Öl-in-Wasser-, Silikon-in-Wasser-, Wasser-in-Öl-, Wasser-in-Silikon-, Öl-in-Wasser-in-Öl-, Wasser-in-Öl-in-Wasser- Emulsion können einen emulgatoren enthalten.

Von dem Fachmann bekannt, hängt der Auswahl des Emulgators von der Darreichungsform der erfindungsgemäßen Zusammensetzungen ab. So enthalten erfindungsgemäße Öl-in-Wasser-Emulsionen (O/W) bevorzugt mindestens einen Emulgator mit einem HLB-Wert > 7 und gegebenenfalls einen Coemulgator. Die Wasser-in-Öl- (W/O) oder Wasser-in-Silikon-Emulsionen (W/Si) enthalten bevorzugt die ein oder mehrere Silikonemulgatoren (W/S) mit einem HLB-Wert ≤ 8 oder ein oder mehrere W/O-Emulgatoren mit einem HLB-Wert < 7 und gegebenenfalls ein oder mehrere O/W-Emulgatoren mit einem HLB-Wert > 10 enthalten.

O/W Emulgatoren können vorteilhaft ausgewählt werden aus der Gruppe der nichtionischen, anionischen, kationischen oder amphoteren Emulgatoren.

Unter den nichtionischen Emulgatoren befinden sich:
a) Partialfettsäureester und Fettsäureester mehrwertiger Alkohole und deren ethoxylierte Derivate;
b) Ethoxylierte Fettalkohole und Fettsäuren;
c) Ethoxylierte Fettamine, Fettsäureamide, Fettsäurealkanolamide;
d) Alkylphenolpolyglycolether (z.B. Triton X); und
e) Ethoxylierte Fettalkoholether

Besonders vorteilhafte nichtionische O/W-Emulgatoren sind ethoxylierte Fettalkohole oder Fettsäuren, bevorzugt PEG-100-Stearat, PEG-40-Stearat, PEG-50 stearate , Ceteareth-20, Ceteth-20, Steareth-20, Ceteareth-12, Ceteth-12, Steareth-12, Ester aus Mono-, Oligo- oder Polysacchariden mit Fettsäuren, bevorzugt Cetearylglucosid, Methylglucosedistearat, Glycerylmonostearate (selbstemulgierend),Sorbitanester wie zum Beispiel Sorbitanstearate (Tween^{®} 20 und Tween^{®} 60 der Firma Uniqema) , Sorbitanpalmitate (span 40, Uniqema), Glycerylstearylcitrate, Sucroseester wie zum beispiel Sucrosestearate, PEG-20 methylglucose sequistearate) Dicarbonsäureester von Fettalkohol (Dimyristyltartrate).

Vorteilhafte anionische Emulgatoren sind Seifen (z. B. Natrium- oder Triethanolamin-Salze der Stearin- oder Palmitinsaeure), Ester der Zitronensäure wie Glycerylstearatcitrat, Fettalkoholsulfate sowie Mono-, Di- und Trialkylphosphosäureester und deren Ethoxylate.

Unter den kationischen Emulgatoren befinden sich quaternäre Ammoniumverbindungen mit einem langkettigen aliphatischen Rest z.B. Distearyldimonium Chloride.

Unter den amphoteren Emulgatoren befinden sich:
a) Alkylamininoalkancarbonsäuren;
b) Betaine, Sulfobetaine; und
c) Imidazolinderivate

Weiterhin gibt es natürlich vorkommende Emulgatoren, zu denen Bienenwachs, Wollwachs, Lecithin und Sterole gehören.

Die Silikonemulgatoren können vorteilhaft aus der Gruppe enthaltend Alkyldimethiconcopolyole wie z. B. Cetyl PEG/PPG 10/1 Dimethiconcopolyol (ABIL^{®} EM 90 der Fa. Evonik) oder Lauryl PEG/PPG-18/18 Dimethicone (Dow Corning^{®} 5200 Formulation Aid der Fa. Dow Corning Ltd.) und Dimethiconecopolyole wie z. B. PEG-10 Dimethicone (KF-6017 der Fa. Shin Etsu), PEG/PPG-18/18 Dimethicone (Dow Corning Formulation aid 5225C der Fa. Dow Corning Ltd.), PEG/PPG-19/19 Dimethicone (Dow Corning BY-11 030 der Fa. Dow Corning Ltd.) oder Trimethylsilylamodimethicone gewählt werden.

Die W/O-Emulgatoren mit einem HLB-Wert < 7 können vorteilhaft aus der Gruppe Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 bis 18 C-Atomen, Propylenglycolester gesättigter und/oder ungesättigter, verzweigter und/oder unverzweigter Alkancarbonsäuren einer Kettenlänge von 8 bis 24, insbesondere 12 bis 18 C-Atomen sowie Sorbitanester gesättigter und/oder ungesättigter, verzweigter und/oder unverzweigter Alkancarbonsäuren einer Kettenlänge von 8 bis 24, insbesondere 12 bis 18 C-Atomen.

Besonders vorteilhafte *W*/*O-Emulgatoren* sind :Glycerylmonostearat" Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol,Chimylalkohol, Polyethylenglycol(2)stearylether (Steareth-2), Glycerylmonolaurat, Glycerylmonocaprinat und Glycerylmonocaprylat.

Weitere mögliche W/O-Emulgatoren warden gewählt aus der Gruppe der Verbindungen Polyglyceryl-2-dipolyhydroxystearat, PEG-30 Dipolyhydroxystearat, Cetyl Dimethicon Copolyol, Polyglyceryl-3 Diisostearat enthält.

Die O/W-Emulgatoren mit einem HLB-Wert > 10 können vorteilhaft aus der Gruppe enthaltend Lecithin, Trilaureth-4-Phosphat, Polysorbate-20, Polysorbate-60, PEG-22-Dodecylglycol Copolymer, Sucrosestearat und Sucroselaurat gewählt werden.

Als geeignete Coemulgatoren für die erfindungsgemässen O/W-Emulsionen können Fettalkohole mit 8 bis 30 Kohlenstoffatomen, Monoglycerinester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, Propylenglycolester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, sowie Sorbitanester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, eingesetzt werden.

Besonders vorteilhafte Coemulgatoren sind Glycerylmonostearat, Glycerylmonooleat, Diglycerylmonostearat, Sorbitanmonoisostearat, Saccharosedistearat, Cetylalkohol, Stearylalkohol, Behenylalkohol, Isobehenylalkohol und Polyethylenglycol(2)stearylether (Steareth-2).

### UV- Filter:

Die erfindungsgemäßen Zusammensetzungen können Sonnenschutzfilter enthalten, wobei die Gesamtmenge der Sonnenschutzfilter 0 Gew.-% bis 30 Gew.-%, vorteilhaft 0 Gew.-% bis 20 Gew.-%, besonders vorteilhaft 0 Gew.-% bis 10 Gew.-%, jeweils bezogen auf das Gesamtgewicht der erfindungsgemäßen Zusammensetzung, beträgt. Die Sonnenschutzfilter (oder UV-Filter) können unter den organischen Filtern, den physikalischen Filtern und deren Gemischen ausgewählt werden.

Die erfindungsgemäßen Zusammensetzungen können UV-A-, UV-B-Filter oder Breitbandfilter enthalten. Die eingesetzten organischen UV-Filter können öllöslich oder wasserlöslich sein. Die untere beigefügte Liste der genannten UV-Filter ist selbstverständlich nicht limitierend.

Von den UV-B-Filtern sind beispielsweise zu nennen:
(1) Salicylsäurederivate, besonders Homomenthylsalicylat, Octylsalicylat und Salicylsäure(4-isopropylbenzyl)ester;
(2) Zimtsäurederivate, insbesondere 2-Ethylhexyl-p-methoxycinnamat, das von der Firma DSM unter der Bezeichnung Parsol MCX^{®} erhältlich ist und 4-Methoxyzimtsäureisopentylester;
(3) flüssige β,β'-Diphenylacrylatderivate, insbesondere 2-Ethylhexyl-α,β'-diphenylacrylat oder Octocrylen, das von der Firma BASF unter der Bezeichnung UVINUL N539^{®} erhältlich ist;
(4) p-Aminobenzoesäurederivate, insbesondere 4-(dimethylamino)-benzoesäure (2-ethylhexyl)-ester, 4-(Dimethylamino)benzoesäureamylester;
(5) 3-Benzylidencampher-Derivate, insbesondere 3-(4-Methylbenzyliden)campher der von der Firma Merck unter der Bezeichnung EUSOLEX 6300^{®} im Handel ist, 3-Benzylidencampher, Benzylidencamphersulfonsäure und Polyacrylamidomethyl-Benzylidencampher;
(6) 2-Phenylbenzimidazol-5-sulfonsaeure, die unter der Bezeichnung EUSOLEX 232^{®} von Merck erhältlich ist;
(7) 1,3,5-Triazinderivate, insbesondere:
   - 2,4,6-Tris[p-(2'-ethylhexyl-1'-oxycarbonyl)anilino]-1,3,5-triazin, das von der Firma BASF unter der Bezeichnung UVINUL T150^{®} angeboten wird, und
   - Dioctylbutamidotriazon, das von der Firma Sigma 3V unter der Bezeichnung UVASORB HEB^{®} angeboten wird;
(8) Ester der Benzalmalonsäure, insbesondere 4-Methoxybenzalmalonsäuredi-(2-ethylhexyl)ester und 3-(4-(2,2-Bisethoxycarbonylvinyl)-phenoxy)propenyl)-methoxysiloxan/ dimethylsiloxan-Copolymer, das von der Firma DSM unter der Bezeichnung Parsol^{®} SLX erhältlich ist; und
(9) die Gemische dieser Filter.

Als UV-A-Filter sind beispielsweise zu nennen:
(1) Dibenzoylmethanderivate, besonders das 4-(t-Butyl)-4'-methoxydibenzoylmethan, das von der Firma DSM unter der Bezeichnung PARSOL 1789^{®} angeboten wird, und 1-Phenyl-3-(4'-isopropylphenyl)propan-1,3-dion;
(2) Benzol-1,4-[di(3-methylidencampher-10-sulfonsaeure)], gegebenenfalls ganz oder teilweise neutralisiert, welches unter der Bezeichnung MEXORYL SX^{®} von Chimex im Handel ist;
(3) 2-(4'-Diethylamino-2'-hydroxybenzoyl)-benzoesäurehexylester (auch Aminobenzophenon);
(4) Silanderivate oder Polyorganosiloxane mit Benzophenongruppen;
(5) Anthranilate, besonders Menthylanthranilat, das von der Firma Symrise unter der Bezeichnung NEO HELIOPAN MA^{®} angeboten wird;
(6) Verbindungen, die pro Molekül mindestens zwei Benzoazolylgruppen oder mindestens eine Benzodiazolylgruppe enthalten, insbesondere 1,4-Bis-benzimidazolylphenylen-3,3',5,5'-tetra-sulfonsaeure sowie ihre Salze, die von der Firma Symrise im Handel sind;
(7) Siliciumderivate von Benzimidazolylbenzazolen, die N-substituiert sind, oder von Benzofuranylbenzazolen, insbesondere:
   - 2-[1-[3-[1,3,3,3-Tetramethyl-1-[(trimethylsilyl)oxy]disiloxanyl]-propyl]-1H-benzimidazol-2-yl]-benzoxazol;
   - 2-[1-[3-[1,3,3,3-Tetramethyl-1-[(trimethylsilyl)oxy]disiloxanyl]-propyl]-1H-benzimidazol-2-yl]-benzothiazol;
   - 2-[1-(3-Trimethylsilanylpropyl)-1H-benzimidazol-2-yl]-benzoxazol;
   - 6-Methoxy-1,1'-bis(3-trimethylsilanylpropyl)1H,1'H-[2,2']dibenzimidazolylbenzoxazol;
   - 2-[1-(3-Trimethylsilanylpropyl)-1H-benzimidazol-2-yl]-benzothiazol; die in der Patentanmeldung EP-A-1 028 120 beschrieben sind;
(8) Triazinderivate, insbesondere 2,4-bis-[5-1 (Dimethylpropyl)benzoxazol-2-yl-(4-phenyl)-imino]-6-(2-ethylhexyl)-imino-1,3,5-triazin, das von der Firma 3V unter der Bezeichnung Uvasorb^{®}K2A angeboten wird; und
(9) deren Gemische.

Als Breitbandfilter sind beispielweise zu nennen :
(1) Benzophenonderivate, beispielsweise
   - 2,4-Dihydroxybenzophenon (Benzophenon-1);
   - 2,2',4,4'-Tetrahydroxybenzophenon (Benzophenon-2);
   - 2-Hydroxy-4-methoxybenzophenon (Benzophenon-3), welches von der Firma BASF unter der Bezeichnung UNIVNUL M40^{®} erhältlich;
   - 2-Hydroxy-4-methoxybenzophenon-5-sulfonsäure (Benzophenon-4), sowie ihre Sulfonatform (Benzonphenon-5), welche von der Firma BASF unter der Bezeichnung UVINUL MS40^{®} erhältlich sind;
   - 2,2'-Dihydroxy-4,4'-dimethoxybenzophenon (Benzophenon-6);
   - 5-Chlor-2-hydroxybenzophenon (Benzophenon-7);
   - 2,2'-Dihydroxy-4-methoxybenzophenon (Benzophenon-8);
   - das Dinatriumsalz von 2,2'-Dihydroxy-4,4'-dimethoxybenzophenon-5,5'-disulfonsäure (Benzophenon-9);
   - 2-Hydroxy-4-methoxy-4'-methylbenzophenon (Benzophenon-10);
   - Bis-(2,4-dihydroxyphenyl)methanon (Benzophenon-11); und
   - 2-Hydroxy-4-(octyloxy)-benzophenon (Benzophenon-12).
(2) Triazinderivate, insbesondere das 2,4-Bis{[4-2-ethylhexyloxy)-2-hydroxy]-phenyl}-6-(4-methoxyphenyl)-1,3,5-triazin, das von der Firma BASF unter der Bezeichnung TINOSORB S^{®} angeboten wird, und das 2,2'-Methylen-bis[6-(2H-benzotriazol-2-yl)4-(1,1,3,3-tetramethylbutyl)phenol], das von der Firma BASF unter der Bezeichnung TINOSORB M^{®} erhältlich ist; und
(3) 2-(1H-benzotriazol-2-yl)-4-methyl-6-[2-methyl-3-[1,3,3,3-tetramethyl-1-[(trimethylsilyl)oxy]disiloxanyl]propyl]-phenol mit der INCI-Bezeichnung Dromotrizole Trisiloxane.

Es können auch ein Gemisch von mehreren Filtern und ein Gemisch von UV-B-Filtern, UV-A-Filtern und Breitbandfilter sowie Gemische mit physikalischen Filtern verwendet werden.

Von den physikalischen Filtern können die Sulfat des Bariums, Oxide von Titan (Titandioxid, amorph oder kristallin in Form von Rutil und/oder Anatas), von Zink, von Eisen, von Zirconium, von Cer, Silicium, Mangan oder deren Gemische angegeben werden. Die Metalloxide können in Partikelform mit einer Grösse im Mikrometerbereich oder Nanometerbereich (Nanopigmente) vorliegen.

Die mittleren Partikelgrössen betragen für die Nanopigmente beispielsweise 5 bis 100 nm.

Farbstoffe:
Gegebenenfalls enthalten die erfindungsgemäßen Zusammensetzungen einen Farbstoff, der aus der Gruppe von lipophilen Farbstoffen, hydrophilen Farbstoffen, Pigmenten und Perlmutt ausgewählt wird. Erfindungsgemäß besonders vorteilhaft ist die Konzentration von Farbstoffen 0 bis 40 Gewichts-%, besonders vorteilhaft 0 bis 30 Gewichts-%, ganz besonders vorteilhaft von 0 bis 25 Gewichts-%, jeweils bezogen auf das Gesamtgewicht der Zusammensetzung.

Beispielweise können die lipophilen Farbstoffe Sudan I (Gelb), Sudan II (Orange), Sudan III (Rot), Sudan IV (Scharlachrot), DC Red 17, DC Green 6, β-Carotin, Sojaöl, DC Yellow 11, DC Violet 2, DC Orange 5 und DC Yellow 10.

Die Pigmente können anorganische oder organische Pigmente sein, die in kosmetische oder dermatologische Zusammensetzung verwendet werden können. Die erfindungsgemäßen verwendeten Pigmente können weiß oder farbig sein, umgehüllt mit einem hydrophoben Behandlungsmittel oder nicht sein.

Vorteilhaft werden die Pigmente gewählt aus der Gruppe der Metalloxide, wie die Oxide von Eisen (insbesondere die Oxide von gelber, roter, brauner, schwarzer Farbe), Titandioxid, Zinkoxid, Ceroxid, Zirconiumoxid, Chromoxid; Manganviolett, Ultramarinblau, Preussisch Blau, Ultramarin und Eisenblau, Bismutoxidchlorid, Perlmutt, mit Titan oder Bismutoxidchlorid ueberzogene Glimmer-Pigmente, farbige Perlglanzpigmente, beispielsweise Titan-Glimmer-Pigmente mit Eisenoxiden, Titan-Glimmer-Pigmente insbesondere mit Eisenblau oder Chromoxid, Titan-Glimmer-Pigmente mit einem organischen Pigment vom vorgenannten Typ, sowie Perlglanzpigmente auf der Basis von Bismutoxidchlorid, Russ, die Pigmente vom Typ D & C und die Lacke auf der Basis von Cochenillerot, Barium, Strontium, Calcium und Aluminium und deren Gemische.

Besonders vorteilhaft werden die Pigmente von Eisenoxiden oder Titandioxid verwendet.

Für eine bessere Benetzbarkeit der Pigmente durch die Öle der Fettphase kann die Oberfläche der Pigmente mit einem hydrophoben Behandlungsmittel behandelt werden. Das hydrophobe Behandlungsmittel wird ausgewählt aus der Gruppe der Siliconen, wie Methiconen, Dimethiconen, Perfluoralkylsilanen; Fettsäuren wie Stearinsäure; Metallseifen, wie Aluminiumdimyristat, dem Aluminiumsalz von hydriertem Talgglutamat, Perfluoralkylphosphaten, Perfluoralkylsilanen, Perfluoralkylsilazanen, Hexafluorpropylenpolyoxiden, Polyorganosiloxanen, die Perfluoralkylperfluorpolyethergruppen enthalten, Aminosäuren; N-acylierten Aminosäuren oder deren Salzen; Lecithin, Isopropyltriisostearyltitanat und deren Gemischen ausgewählt sein. Die N-acylierten Aminosaeuren können eine Acylgruppe mit 8 bis 22 Kohlenstoffatomen enthalten, beispielsweise 2-Ethylhexanoyl, Caproyl, Lauroyl, Myristoyl, Palmitoyl, Stearoyl oder Cocoyl. Die Salze dieser Verbindungen können Aluminiumsalze, Magnesiumsalze, Calciumsalze, Zirkoniumsalze, Zinnsalze, Natriumsalze oder Kaliumsalze sein. Bei der Aminosäure kann es sich beispielsweise um Lysin, Glutaminsäure oder Alanin handeln.

### Konditionierungsmittel:

Gegebenfalls enthalten die erfindungsgemäßen Zusammensetzungen ein Konditionierungsmittel. Erfindungsgemäß bevorzugte Konditionierungsmittel sind beispielsweise alle Verbindungen, welche im International Cosmetic Ingredient Dictionary and Handbook (Volume 3, Herausgeber:T. E. Gottschlack, J. E. Bailey, The Personal Care Products Council, 13. Auflage, 2010) unter Section 3 unter den Stichworten Hair Conditioning Agents, Humectants, Skin-Conditioning Agents, SkinConditioning Agents-Emollient, Skin-Conditioning Agents-Humectant, SkinConditioning Agents-Miscellaneous, Skin-Conditioning Agents-Occlusive und Skin Protectans aufgeführt sind sowie alle in der EP-A 934 956 (5.11-13) unter "water soluble conditioning agent" und "oil soluble conditioning agent" aufgeführten Verbindungen.

Besondere vorteilhafte Konditionierungsstoffe stellen beispielsweise die nach INCI als Polyquaternium bezeichneten Verbindungen dar (insbesondere Polyquaternium-1 bis Polyquaternium-56).

Zu den geeigneten Konditionierungsmitteln zählen beispielsweise auch polymere quaternäre Ammoniumverbindungen, kationische Cellulosederivate, Chitosanderivate Guar Gum Derivate und Polysaccharide, insbesondere Guar Hydroxypropylammoniumchlorid (z.B. Jaguar^{®} Excel, Jaguar^{®}162 der Firma Rhodia).

Weitere erfindungsgemäß vorteilhafte Konditionierungsmittel stellen nichtionische Poly-N-vinylpyrrolidon/Polyvinylacetat-Copolymere (z.B. Luviskol^{®}VA 64 der Firma BASF AG), anionische Acrylat-Copolymere (z.B. Luviflex^{®}Soft der Firma BASF AG), und/oder amphotere Amid/Acrylat/Methacrylat Copolymere (z.B. Amphomer^{®} der Firma National Starch dar. Weitere mögliche Konditioniermittel sind quatemisierte Silikone.

### Tenside:

Die erfindungsgemäßen Zusammensetzungen können auch Tenside enthalten, die aus der Gruppe von anionischen, kationischen, nichtionischen und/oder amphoteren Tensiden ausgewählt werden.

Vorteilhafte anionische Tenside im Sinne der vorliegenden Erfindung sind:
● Acylaminosäuren und deren Salze, wie Acylglutamate, insbesondere Natriumacylglutamat und Sarcosinate, beispielsweise Myristoyl Sarcosin, TEA-Lauroyl Sarcosinat, Natriumlauroylsarcosinat und Natriumcocoylsarkosinat;
● Sulfonsäuren und deren Salze, wie Acylisethionate, beispielsweise Natriumoder Ammoniumcocoylisethionat, Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate;
● Schwefelsäureester, wie Alkylethersulfat, beispielsweise Natrium-, Ammonium-, Magnesium-, MIPA-, TIPALaurethsulfat, Natriummyrethsulfat und Natrium C12-13 Parethsulfat, und Alkylsulfate, beispielsweise Natrium-, Ammoniumund TEA-Laurylsulfat;
● Taurate, beispielsweise Natriumlauroyltaurat und Natriummethylcocoyltaurat,
● Ether-Carbonsäuren, beispielsweise Natriumlaureth-13 Carboxylat und Natrium PEG-6 Cocamide Carboxylat, Natrium PEG-7-Olivenöl-Carboxylat;
● Phosphorsäureester und Salze, wie beispielsweise DEA-Oleth-10 Phosphat und Dilaureth-4 Phosphat;
● Alkylsulfonate, beispielsweise Natriumcocosmonoglyceridsulfat, Natrium C12 bis CI4-Olefinsulfonat, Natriumlaurylsulfoacetat und Magnesium PEG-3 Cocamidsulfat;
● Acylglutamate wie Di-TEA-palmitoylaspartat und Natrium Caprylic/Capric Glutamat,
● Acylpeptide, beispielsweise Palmitoyl hydrolysiertes Milchprotein, Natrium Cocoyl hydrolysiertes Sojaprotein und Natriun/Kalium Cocoyl hydrolysiertes Kollagen;
● Carbonsäuren und Derivate, wie beispielsweise Laurinsäure, Aluminiumstearat, Magnesiumalkanolat und Zinkundecylenat, Ester-Carbonsäuren, beispielsweise Calciumstearoyilactylat, Laureth-6 Citrat und Natrium PEG-4 Lauramidcarboxylat; und
● Alkylarylsulfonate.

Vorteilhafte kationische Tenside im Sinne der vorliegenden Erfindung sind quatemäre Tenside. Quaternäre Tenside enthalten mindestens ein N-Atom, das mit 4 Alkyloder Arylgruppen kovalent verbunden ist. Vorteilhaft sind beispielsweise Alkylbetain, Alkylamidopropylbetain und Alkylamidopropylhydroxysultain.

Weitere vorteilhafte kationische Tenside im Sinne der vorliegenden Erfindung sind ferner Alkylamine, Alkylimidazole und ethoxylierte Amine und insbesondere deren Salze.

Vorteilhafte amphotere Tenside im Sinne der vorliegenden Erfindung sind Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat, Natriumacylamphopropionat, und N-KokosfettsäureamidoethylN-hydroxyethylglycinat Natriumsalze.

Weitere vorteilhafte amphotere Tenside sind N-Alkylaminosäuren, beispielsweise Aminopropylalkylglutamid, Alkylaminopropionsäure, Natriumalkylimidodipropionat und Lauroamphocarboxyglycinat.

Vorteilhafte aktive nicht-ionische Tenside im Sinne der vorliegenden Erfindung sind Alkanolamide, wie Cocamide MEA/DEA/MIPA, Ester, die durch Veresterung von Carbonsäuren mit Ethylenoxid, Glycerin, Sorbitan oder anderen Alkoholen entstehen, Ether, beispielsweise ethoxylierte Alkohole, ethoxyliertes Lanolin, ethoxylierte Polysiloxane, propoxylierte POE Ether, Alkylpolyglycoside wie Laurylglucosid, Decylglycosid und Cocoglycosid, Glycoside mit einem HLB-Wert von wenigstens 20 (z.B. Belsil^{®}SPG 128V der Firma Wacker).

Weitere vorteilhafte nicht-ionische Tenside sind Alkohole und Aminoxide, wie Cocoamidopropylamin-Oxid.

Unter den Alkylethersulfaten sind insbesondere Natriumalkylethersulfate auf Basis von zweioder dreifach ethoxyliertem Laurylund Myristylalkohol bevorzugt. Sie übertreffen die Alkylsulfate deutlich bezüglich der Unempfindlichkeit gegen Wasserhärte, der Verdickbarkeit, der Kältelöslichkeit und insbesondere der Hautund Schleimhautverträglichkeit. Laurylethersulfat weist bessere Schaumeigenschaften als Myristylethersulfat auf, ist diesem aber in der Milde unterlegen.

Alkylethercarboxylate mit mittlerem und besonders mit höherem gehören zu den mildesten Tensiden überhaupt, zeigen aber ein schlechtes Schaumund Viskositätsverhalten. Sie werden oft in Kombination mit Alkylethersulfaten und amphoteren Tensiden eingesetzt.

Sulfobemsteinsäureester (Sulfosuccinate) sind mild und gut schäumende Tenside werden aber wegen ihrer schlechten Verdickbarkeit bevorzugt nur zusammen mit anderen anionischen und amphoteren Tensiden und wegen ihrer geringen Hydrolysestabilität bevorzugt nur in neutralen bzw. gut gepufferten Produkten eingesetzt.

Amidopropylbetaine weisen eine ausgezeichnete Haut- und Augenschleimhautverträglichkeit auf. In Kombination mit anionischen Tensiden lässt sich deren Milde synergistisch verbessern. Bevorzugt ist die Verwendung von Cocamidopropylbetain.

Arnphoacetate/Amphodiacetate besitzen als amphotere Tenside eine sehr gute Hautund Schleimhautverträglichkeit und können konditionierend wirken bzw. die Pflegewirkung von Zusatzstoffen erhöhen. Sie werden ähnlich wie die Betaine zur Optimierung von Alkylethersulfat-Formulierungen eingesetzt. Am meisten bevorzugt sind Natriumcocoamphoacetat und Dinatriumcocoamphodiacetat.

Alkylpolyglykoside sind mild, haben gute Universaleigenschaften, schäumen jedoch schwach. Aus diesem Grund werden sie bevorzugt in Kombinationen mit anionischen Tensiden verwendet.

### Filmbildner:

Vorteilhaft werden der oder die Filmbildner gewählt aus der Gruppe der wasserlöslichen oder wasserdispergierbaren Polyurethane, der Polyharnstoffe, Silikonharze und/oder Polyester sowie der nichtionischen, anionischen, amphoteren und/oder kationischen Polymere und ihren Mischungen.

Vorteilhafte nichtionische Polymere, die in erfindungsgemäßen Zusammensetzungen alleine oder im Gemisch, vorzugsweise auch mit anionischen und/oder amphoteren und/oder zwitterionischen Polymeren enthalten sein können, sind ausgewählt aus:
- Polyalkyloxazoline;
- Vinylacetat Homo- oder Copolymerisate. Hierzu gehören beispielweise Copolymerisate aus Vinylacetat und Acrylsäureester, Copolymerisate aus Vinylacetat und Ethylen, Copolymerisate aus Vinylacetat und Maleinsäureester;
- Acrylsäureester Copolymerisate wie z. B. die Copolymerisate aus Alkyl-Acrylat und Alkyl-Methacrylat, Copolymerisate aus Allcyl-Acrylat und Urethanen;
- Copolymerisate aus Acrylnitril und nichtionischem monomer ausgewählt aus Butadien und (Meth)-Acrylat;
- Styrol Homo- und Copolymerisate. Hierzu gehören beispielweise Homopolystyrol, Copolymerisate aus Styrol und Alkyl-(Meth)Acrylat , Coplymerisate aus Styrol, Alkyl-Methacrylat und Allcyl-Acrylat, Copolymerisate aus Styrol und Butadien, Copolymerisate aus Styrol, Butadien und Vinylpyridin;
- Polyamide;
- Vinyllactame Homo- oder Copolymere, wie Vinylpyrrolidon-Homo- oder Copolymerisate;
   hierzu gehören beispielweise Polyvinylpyrrolidon, Polyvinylcaprolactam, Copolymerisate aus N-Vinylpyrrolidon und Vinylacetat und/oder Vinylpropionat in verschiedenen Konzentrationsverhältnissen, Polyvinylcaprolactam, Polyvinylamide und deren Salze sowie Copolymere aus Vinylpyrrolidon und Dimethylaminoethyl-methacrylat, Terpolymere aus Vinylcaprolactam, Vinylpyrrolidon und Dimethylaminoethylmethacrylat;
- Polysiloxane;
- Homopolymere des N-Vinylformamids z. B. PVF von National Starch.

Besondere bevorzugte nichtionische Polymere sind Acrylsäureester Copolymerisate, Homopolymere und Copolymere des Vinylpyrrolidons und Polyvinylcaprolactam.

Ganz besondere bevorzugte nichtionische Polymere sind Homopolymere des Vinylpyrrolidons z. B. Luviskol® K von der Firma BASF, Copolymerisate aus Vinylpyrrolidon und Vinylacetat z. B. Luviskol® VA Typen der Firma BASF oder PVPVA® S630L der Firma ISP, Terpolymere aus Vinylpyrrolidon, Vinylacetat und Propionat wie z. B. Luviskol® VAP von BASF und Polyvinylcaprolactame z.B. Luviskol® PLUS der Firma BASF.

Vorteilhafte anionische Polymere sind Homo-oder Copolymere mit Säuregruppen enthaltenden Monomereinheiten, welche gegebenenfalls mit Comonomeren, die keine Säuregruppen enthalten copolymerisiert sind. Geeignete Monomere sind ungesättigte, radikalisch polymerisierbare-Verbindungen, welche mindestens eine Säuregruppe besitzen, insbesondere Carbonsäure, Sulfonsäure oder Phosphonsäure.

Vorteilhafte anionische Polymere enthaltend Carbonsäuregruppe sind:
- Acrylsäure oder Methacrylsäure Homo- oder Copolymer oder die Salze davon. Hierzu gehören beispielweise die Copolymerisate aus Acrylsäure und Acrylamide und/oder deren Natriumsalze, Copolymerisate aus Acrylsäure und/oder Methacrylsäure und einem ungesättigten Monomer ausgewählt aus Ethylen, Styrol, Vinylester, Acrylsäureester, Methacrylsäureester, gegebenenfalls ethoxylierten Verbindungen, Copolymerisate aus Vinylpyrrolidone, Acrylsäure und C1-C20 Alkyl Methacrylate z.B. Acrylidone^{®} LM der Firma ISP, Copolymerisate aus Methacrylsäure, Ethylacrylate und Tert-butyl Acrylate z.B. Luvimer^{®} 100 P der Firma BASF, Copolymerisate aus Ethyl Acrylates und Methyl Methacrylates z.B. Daitosol der Firma Daito Kasei Kogyo Co., Copolymerisate aus Ethyl Methacrylates, N-Butyl Acrylates und 2- methylhexyl Acrylates z.B. Daitosol SJ der Firma Daito Kasei Kogyo Co., Copolymerisate aus Acrylates und Vinyl Acetate z.B. Vinylsol 2140 aus der Firma Daito Kasei Kogyo Co., Copolymerisate aus Alkyl (meth)acrylates z.B. Yodosol GH34F von der Firma Nippon LSC Ltd., Copolymerisate aus Acrylates und Ammonium Methacrylate z.B. Ultrasol von der Firma Ganz Chemical, Copolymerisate aus Styrol und Acrylates z.B. Yodosol GH41F aus der Firma Nippon LSC Ltd.,Joncryl 77 von der Firma BASF, Copolymerisate aus Acrylate, Styrol und Ammonium Methacrylates z.B. Syntran 5760 von der Firma Interpolymer, Copolymerisate aus Acrylates und Dimethylaminoethyl Methacrylate von der Firma Interpolymer;
- Crotonsäurederivat-Homo- oder Copolymer oder die Salze davon. Hierzu gehören beispielweise Vinylacetat/Crotonsäure-, Vinylacetat/Acrylat- und/oder Vinylacetat/Vinylneodecanoat/Crotonsäure-Copolymere und Natriumacrylat/Vinylalkohol-Copolymere;
- ungesättigte C4-C8 Carbonsäurederivate oder Carbonsäureanhydrid Copolymer ausgewählt aus Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Vinylester, Vinylether, Vinylhalogenderivate, Phenylvinylderivate, Acrylsäure, Acrylsäureester oder Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Allylester, Methallylester und ggf. Acrylamide, Methacrylamide, alpha-Olefin, Acrylsäureester, Methacrylsäureester, Vinylpyrrolidone. Weitere bevorzugte Polymere sind Methylvinylether/Maleinsäure-Copolymere, die durch Hydrolyse von Vinylether/Maleinsäureanhydrid-Copolymeren entstehen. Diese Polymere können auch teilverestert (Ethyl, Isopropyl- bzw. Butylester) oder teilamidiert sein.
- in Wasser lösliche oder dispergierbare anionische Polyurethane, z. B. Luviset^{®} PUR von BASF, Baycusan^{®} C 1000, Baycusan^{®} C 1001, Baycusan^{®} C 1003 und Baycusan^{®} C 1004 von der Firma Bayer MaterialScience, Avalure UR-405^{®}, Avalure UR-410^{®}, Avalure UR-425^{®}, Avalure UR-450^{®} von der Firma Noveon, die von den erfindungsgemäßen Polyurethanen verschieden sind,
wobei diese Aufstellung selbstverständlich nicht limitierend sein soll.

Vorteilhafte anionische Polymere enthaltend Sulfonsäuregruppe sind Salze von Polyvinylsulfonsäure, Salze von Polystyrolsulfonsäure wie z. B. Natriumpolystyrolsulfonat oder Salze von Polyacrylamidesulfonsäure.

Besonders vorteilhafte anionische Polymere sind Acrylsäure Copolymere, Crotonsäurederivat-Copolymer, Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Vinylester, Vinylether, Vinylhalogenderivate, Phenylvinylderivate, Acrylsäure, Acrylsäureester und Salze von Polystyrolsulfonsäure.

Ganz besondere vorteilhafte anionische Polymere sind Acrylat-Copolymere, z.B. Luvimer von BASF, Covacryl E 14 und Covacryl A 15 von Sensient Cosmetic Technologies, Styrene/ Acrylate/Ammonium Methacrylates z.B. Syntran 5760 von der Firma Interpolymer Ethylacrylat/N-tert.-Butylacrylamid/Acrylsäure-Copolymere ULTRAHOLD^{®} STRONG der Firma BASF, VA/Crotonat/Vinylneodecanoat-Copolymer z. B. Resyn 28-2930 von National Starch, Copolymerisate wie. Z. Copolymerisate aus Methylvinylether und Maleinsäureanhydrid teilverestert z.B. GANTREZ^{®} der Firma ISP und Natrium-Polystyrol-Sulfonate z. B. Flexan 130 von National Starch.

Vorteilhafte amphotere Polymere können unter den Polymeren ausgewählt werden, die statistisch in der Polymerkette verteilte Einheiten A und B enthalten, wobei A eine Einheit bedeutet, die von einem Monomer mit mindestens einem basischen Stickstoffatom abgeleitet ist, und B eine Einheit ist, die von einem sauren Monomer stammt, das eine oder mehrere Carboxygruppen oder Sulfonsäuregruppen aufweist, oder A und B können Gruppen bedeuten, die von zwitterionischen Carboxybetainmonomeren oder Sulfobetainmonomeren abgeleitet sind; A und B können auch eine kationische Polymerkette bedeuten, die primäre, sekundäre, tertiäre oder quartanäre Gruppen enthält, worin mindestens eine Aminogruppe eine Carboxygruppe oder Sulfonsäuregruppe trägt, die über eine Kohlenwasserstoffgruppe gebunden ist, oder B und C sind Teil einer Polymerkette mit Ethylen-α,β-dicarbonsäureeinheit, bei der die Carbonsäuregruppen mit einem Polyamin umgesetzt wurden, das eine oder mehrere primäre oder sekundäre Aminogruppen enthält.

Besondere vorteilhafte amphotore Polymere sind:
- Polymere, die bei der Copolymerisation eines von einer Vinylverbindung mit Carboxygruppe abgeleiteten Monomers, wie insbesondere Acrylsäure, Methacrylsäure, Maleinsäure, α-Chloracrylsäure, und einem basischen Monomer gebildet werden, das von einer Vinylverbindung abgeleitet ist, die substituiert ist und mindestens ein basisches Atom enthält, wie insbesondere Dialkylaminoalkylmethacrylat und -acrylat, Dialkylaminoalkylmethacrylamid und -acrylamid. Solche Verbindungen sind in dem amerikanischen Patent Nr. 3 836 537 beschrieben worden.
- Polymere mit Einheiten, die abgeleitet sind von: a) mindestens einem Monomer, das unter den Acrylamiden oder Methacrylamiden ausgewählt ist, die am Stickstoffatom mit einer Alkylgruppe substituiert sind, b) mindestens einem sauren Comonomer, das eine oder mehrere reaktive Carboxygruppen enthält, und c) mindestens einem basischen Comonomer, wie Estern von Acrylsäure und Methacrylsäure mit primären, sekundären, tertiären und quartären Aminosubstituenten und dem Quaternisierungsprodukt von Dimethylaminoethylmethacrylat mit Dimethylsulfat oder Diethylsulfat.
   Erfindungsgemäß besonders bevorzugte N-substituierte Acrylamide oder Methacrylamide sind Verbindungen, deren Alkylgruppen 2 bis 12 Kohlenstoffatome enthalten, besonders N-Ethylacrylam i d , N-t-Butylacrylamid, N-t-Octylacrylamid, N-Octylacrylamid, N-Decylacrylamid, N-Dodecylacrylamid sowie die entsprechenden Methacrylamide.
   Die sauren Comonomere sind insbesondere unter Acrylsäure, Methacrylsäure, Crotonsäure, Itaconsäure, Maleinsäure, Fumarsäure sowie den Alkylmonoestern mit 1 bis 4 Kohlenstoffatomen von Maleinsäure, Maleinsäureanhydrid, Fumarsäure oder Fumarsäureanhydrid ausgewählt.
   Bevorzugte basische Comonomere sind Aminoethylmethacrylat, Butylaminoethylmethacrylat, N,N-Dimethylaminoethylmethacrylat, N-t-Butylaminoethylmethacrylat.
- Vernetzte und ganz oder teilweise acylierte Polyaminoamide, die von Polyaminoamiden der folgenden allgemeinen Formel abgeleitet sind:

   -[CO-R-CO-Z]-

   worin R eine zweiwertige Gruppe bedeutet, die von einer gesättigten Dicarbonsäure, einer aliphatischen Mono- oder Dicarbonsäure mit ethylenischer Doppelbindung, einem Ester dieser Säuren mit einem niederen Alkanol mit 1 bis 6 Kohlenstoffatomen oder einer Gruppe abgeleitet ist, die bei der Addition einer dieser Säuren an ein bisprimäres oder bis-sekundäres Amin entsteht, und Z eine Gruppe bedeutet, die von einem bis-primären, mono- oder bissekundären Polyalkylenpolyamin abgeleitet ist, und vorzugsweise: a) in Mengenanteilen von 60 bis 100 Mol-% die Gruppen -NH-[(CH₂)ₓ-NH-]p- mit x = 2 und p = 2 oder 3 oder x = 3 und p = 2, wobei diese Gruppe, von Diethylentriamin, Triethylentetramin oder Dipropylentriamin abgeleitet ist; b) in Mengenanteilen von 0 bis 40 Mol-% die Gruppe -NH-[(CH₂)ₓ-NH-]p-, worin x = 2 und p = 1, die von Ethylendiamin abgeleitet ist, oder die Gruppe, die von Piperazin stammt: 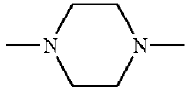 c) in Mengenanteilen von 0 bis 20 Mol-%, die Gruppe -H-(CH₂)₆-NH-, die von Hexamethylendiamin abgeleitet ist, wobei diese Polyaminoamide durch Addition eines bifunktionellen Vernetzungsmittels, das unter den Epihalohydrinen, Diepoxiden, Dianhydriden und bisungesättigten Derivaten ausgewählt ist, in einer Menge von 0,025 bis 0,35 mol Vernetzungsmittel pro Aminogruppe des Polyaminoamids vernetzt und mit Acrylsäure, Chloressigsäure oder einem Alkansulfon oder deren Salzen acyliert ist.
   Die gesättigten Carbonsäuren sind vorzugsweise unter den Säuren mit 6 bis 10 Kohlenstoffatomen ausgewählt, wie Adipinsäure, 2,2,4-Trimethyladipinsäure und 2,4,4,-Trimethyladipinsäure, Terephthalsäure; Säuren mit ethylenischer Doppelbindung, wie beispielsweise Acrylsäure, Methacrylsäure und Itaconsäure.
   Die bei der Acylierung verwendeten Alkansultone sind vorzugsweise Propansulton oder Butansulton, die Salze der Acylierungsmittel sind vorzugsweise die Natriumsalze oder Kaliumsalze.
- Polymeren mit zwitterionischen Einheiten der folgenden Formel: 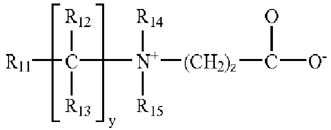 worin R₁₁ eine polymerisierbare ungesättigte Gruppe bedeutet, wie Acrylat, Methacrylat, Acrylamid oder Methacrylamid, y und z ganze Zahlen von 1 bis 3 bedeuten, R₁₂ und R₁₃ ein Wasserstoffatom, Methyl, Ethyl oder Propyl bedeuten, R₁₄ und R₁₅ ein Wasserstoffatom oder eine Alkylgruppe bedeuten, die so gewählt ist, dass die Summe der Kohlenstoffatome R₁₄ und R₁₅ 10 nicht übersteigt.
   Polymere, die solche Einheiten enthalten, können auch Einheiten aufweisen, die von nicht zwitterionischen Monomeren stammen, wie Dimethyl- und Diethylaminoethylacrylat oder Dimethyl- und Diethylaminoethylmethacrylat oder Alkylacrylate oder Alkylmethacrylate, Acrylamide oder Methacrylamide oder Vinylacetat.
- Polymere, die von Chitosan abgeleitet sind und Monomereinheiten enthalten, die den folgenden Formeln entsprechen: 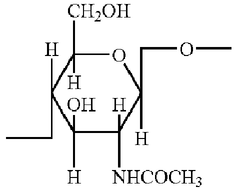 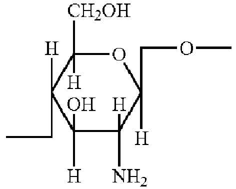 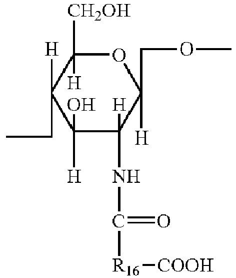 wobei die erste Einheit in Mengenanteilen von 0 bis 30 %, die zweite Einheit in Mengenanteilen von 5 bis 50 % und die dritte Einheit in Mengenanteilen von 30 bis 90 % enthalten ist, mit der Maßgabe, dass in der dritten Einheit R₁₆ eine Gruppe der folgenden Formel bedeutet: 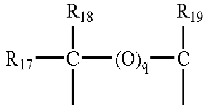 worin bedeuten: falls q = 0, die Gruppen R₁₇, R₁₈ und R₁₉, die gleich oder verschieden sind, jeweils ein Wasserstoffatom, Methyl, Hydroxy, Acetoxy oder Amino, einen Monoalkylaminrest oder einen Dialkylaminrest, die gegebenenfalls durch ein oder mehrere Stickstoffatome unterbrochen und/oder gegebenenfalls mit einer oder mehreren Gruppen Amino, Hydroxy, Carboxy, Alkylthio, Sulfonsäure, Alkylthio, dessen Alkylgruppe einen Aminorest trägt, wobei mindestens eine der Gruppen R₁₇, R₁₈ und R₁₉ in diesem Fall ein Wasserstoffatom bedeutet; oder falls q = 1, die Gruppen R₁₇, R₁₈ und R₁₉ jeweils ein Wasserstoffatom, sowie die Salze, die diese Verbindungen mit Basen oder Säuren bilden.
- Polymere, die der folgenden allgemeinen Formel entsprechen und die beispielsweise in dem französischen Patent 1 400 366 beschrieben sind: 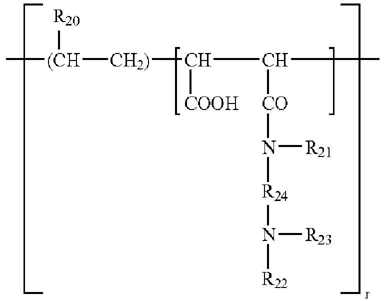 worin R₂₀ ein Wasserstoffatom, CH₃O, CH₃CH₂O oder Phenyl bedeutet, R₂₁ ein Wasserstoffatom oder eine niedere Alkylgruppe, wie Methyl oder Ethyl ist, R₂₂ ein Wasserstoffatom oder eine niedere C₁₋₆-Alkylgruppe bedeutet, wie Methyl oder Ethyl, R₂₃ eine niedere C₁₋₆-Alkylgruppe ist, wie Methyl oder Ethyl oder eine Gruppe der Formel: -R₂₄-N(R₂₂)₂, wobei R₂₄ eine Gruppe -CH₂-CH₂, -CH₂-CH₂-CH₂- oder -CH₂-CH(CH₃)- bedeutet und wobei R₂₂ die oben angegebenen Bedeutungen aufweist.
- Polymere, die bei der N-Carboxyalkylierung von Chitosan gebildet werden können, wie N-Carboxymethylchitosan oder N-Carboxybutylchitosan.
- Amphotere Polymere vom Typ -D-X-D-X, die ausgewählt sind unter:
   a) Polymere, die durch Einwirkung von Chloressigsäure oder Natriumchloracetat auf Verbindungen mit mindestens einer Einheit der folgenden Formel gebildet werden:

      worin D die Gruppe bedeutet 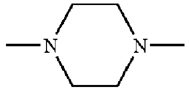 und X die Symbole E oder E' bedeutet, wobei E oder E', die gleich oder verschieden sind, eine zweiwertige Gruppe bedeuten, bei der es sich um eine geradkettige oder verzweigte Alkylengruppe mit bis zu 7 Kohlenstoffatomen in der Hauptkette handelt, die unsubstituiert vorliegt oder mit Hydroxygruppen substituiert ist und ein oder mehrere Sauerstoffatome, Stickstoffatome oder Schwefelatome und 1 bis 3 aromatische und/oder heterocyclische Ringe enthalten kann; wobei die Sauerstoffatome, Stickstoffatome und Schwefelatome in Form der folgenden Gruppen vorliegen: Ether, Thioether, Sulfoxid, Sulfon, Sulfonium, Alkylamin, Alkenylamin, Hydroxy, Benzylamin, Aminoxid, quartäres Ammonium, Amid, Imid, Alkohol, Ester und/oder Urethan.
   b) Polymere der Formel worin D die Gruppe bedeutet 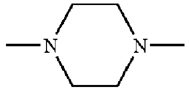 und X das Symbol E oder E' und mindestens einmal E' bedeutet; wobei E die oben angegebenen Bedeutungen aufweist und E' eine zweiwertige Gruppe ist, bei der es sich um eine geradkettige oder verzweigte Alkylengruppe mit bis zu 7 Kohlenstoffatomen in der Hauptkette handelt, die unsubstituiert vorliegt oder mit einer oder mehreren Hydroxygruppen substituiert ist und ein oder mehrere Stickstoffatome enthält, wobei das Stickstoffatom mit einer Alkylgruppe substituiert ist, die gegebenenfalls durch ein Sauerstoffatom unterbrochen ist und zwingend eine oder mehrere Carboxyfunktionen oder eine oder mehrere Hydroxyfunktionen enthält und durch Umsetzung mit Chloressigsäure oder Natriumchloracetat betainisiert ist.
- Alkyl(C₁₋₅)vinylether/Maleinsäureanhydrid-Copolymere, die teilweise durch Semiamidierung mit einem N,N-Dialkylaminoalkylamin wie N,N-Dimethylaminopropylamin oder einem N,N-Dialkylaminoalkohol teilweise modifiziert sind. Diese Polymere können auch weitere Comonomere enthalten, wie Vinylcaprolactam.

Ganz besondere vorteilhafte amphotere Polymere sind z.B. die Copolymere Octylacrylamid/Acrylate/Butylamino-Ethyhnethacrylat-Copolymer, die unter den Bezeichnungen AMPHOMER^{®}, AMPHOMER^{®} LV 71 oder BALANCE^{®} 47 der Firma NATIONAL STARCH im Handel sind, Methacryloyl ethyl betaine/Acrylates copolymer z.B. Diaformer^{®} Z-301N der Firma Mitsubishi Chemical Inc., Acrylates/Lauryl Acrylate/Stearyl Acrylates/Ethylamine Oxide Methacrylate Copolymer Z.B. Diaformer^{®} Z-711, Diaformer^{®} Z-712, Diaformer^{®} Z-731 und Diaformer^{®} Z-651 der Firma Mitsubishi Chemical Inc. und Methylmethacrylat/Methyldimethylcarboxymethylammoniumethylmethacrylat-Copolymere.

### Lösungsmittel:

Das kosmetische akzeptable Medium der erfindungsgemäßen Zusammensetzungen kann Wasser und gegebenenfalls ein kosmetisch im Wasser mischbares geeignetes organisches Lösungsmittel sein.

Das verwendete Wasser in den erfindungsgemäßen Zusammensetzungen kann ein Blütenwasser, reines demineralisiertes Wasser, Mineralwasser, Thermalwasser und/oder Meerwasser sein.

Die bevorzugten Lösungsmittel sind beispielsweise die aliphatischen Alkohole mit C1-4 Kohlenstoffatomen wie Ethanol und Isopropanol; Polyol und deren Derivate wie Propylenglykol, Dipropylenglykol, Butylen-1,3-glykol, Polypropylenglykol, Glykolether wie Alkyl (C1-4)ether von Mono-, Di- oder Tripropylenglykol oder Mono-; Di- oder Triethylenglykol, und deren Gemischen ausgewählt.

### Treibgase:

Gegebenfalls enthalten die erfindungsgemäßen Treibgase. Die erfindungsgemäßen bevorzugten Treibgase sind Kohlenwasserstoffe wie Propan, Isobutan und n-Butan sowie deren Mischungen. Aber auch Druckluft, Kohlendioxid, Stickstoff, Stickstoffdioxid und Dimethylether sowie Mischungen all dieser Gase sind erfindungsgemäß vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW) wie z.B. 1,2-difluoroethan (Treibmittel 152 A).

### Wirkstoffe:

Die erfindungsgemäßen kosmetischen Zusammensetzungen enthalten zweckmäßig einen oder mehrere kosmetisch wirksame, gegebenenfalls auch pharmazeutisch wirksamen Inhaltsstoffe.

Beispiele kosmetisch gegebenenfalls auch therapeutisch wirksamer Inhaltsstoffe schließen ein: Antiaknemittel, antimikrobielle Mittel, Antitranspirationsmittel, astringierende Mittel, desodorierende Mittel, Konditionierungsmittel für die Haut, hautglättende Mittel, Mittel zur Steigerung der Hauthydratation wie z. B. Glycerin oder Harnstoff (so genannte Feuchhaltmittel), Keratolytika, Radikalfänger für freie Radikale, antiseptische Wirkstoffe, Wirkstoffe gegen Hautalterung und/oder Mittel, welche die Differenzierung und/oder Proliferation und/oder Pigmentierung der Haut modulieren, Vitamine wie Vitamin C, Wirkstoffe mit reizender Nebenwirkung, wie alpha-Hydroxysäuren, β-Hydroxysäuren, alpha-Ketosäuren, β-Ketosäuren, Retinoide (Retinol, Retinal, Retin-Säure) Anthralinen (Dioxyanthranol), Anthranoide, Peroxide (insbesondere Benzoylperoxid), Minoxidil, Lithiumsalze, Antimetabolite, Vitamin D und seine Derivate; Katechine, Flavonoide, Ceramide, Fettsubstanzen, synthetische Öle, Mineralöle, wie Paraffinöle oder Vaselineöle, Siliconöle, Pflanzenöle wie Kokosöl, Süßmandelöl, Aprikosenöl, Maisöl, Jojobaöl, Olivenöl, Avocadoöl, Sesamöl, Palmöl, Eukalyptusöl, Rosmarinöl, Lavendelöl, Kiefernöl, Thymianöl, Minzöl, Kardamonöl, Orangenblütenöl, Sojaöl, Kleieöl, Reisöl, Rapsöl und Rizinusöl, Weizenkeimöl und daraus isoliertes Vitamin E, Nachtkerzenöl, Pflanzenlecithine (z.B. Sojalecithin), aus Pflanzen isolierte Sphingolipide/Ceramide, tierische Öle oder Fette, wie Talg, Lanolin, Butteröl, Fettsäureester, Ester von Fettalkoholen und Wachse mit einem der Hauttemperatur entsprechenden Schmelzpunkt (tierische Wachse, wie Bienenwachs, Carnaubawachs und Candelillawachs, mineralische Wachse, wie mikrokristalline Wachse, und synthetische Wachse, wie Polyethylen- oder Silikonwachse), sowie sämtliche für kosmetische Zwecke geeigneten Öle, wie beispielsweise in dem International Cosmetic Ingredient Dictionary and Handbook (Volume 3, Herausgeber:T. E. Gottschlack, J. E. Bailey, The Personal Care Products Council, 13. Auflage, 2010) erwähnt, mehrfach ungesättigte Fettsäuren, essentielle Fettsäuren (z.B. gamma-Linolensäure), Enzyme, Coenzyme, Enzymhemmstoffe, hydratisierende Mittel, hautberuhigende Mittel, Detergentien oder schaumbildende Mittel, und anorganische oder synthetische mattierende Füllstoffe, mit Ausnahme der nicht vorteilhaften oben genannten nicht-verfilmenden Füllstoffe, abrasive Mittel.

Weiterhin können die erfindungsgemäßen kosmetischen Zusammensetzungen Pflanzenwirkstoffextrakte bzw. daraus gewonnene Auszüge oder Einzelstoffe erwähnt werden enthalten sein, wie solche, die ausgewählt werden aus der Gruppe bestehend aus festen Pflanzenextrakten, flüssigen Pflanzenextrakten, hydrophilen Pflanzenextrakten, lipophilen Pflanzenextrakten, einzelnen Pflanzeninhaltsstoffen; sowie deren Mischungen, wie Flavonoide und ihre Aglyka: Rutin, Quercetin, Diosmin, Hyperosid, (Neo)hesperidin, Hesperitin, Ginkgo Biloba (z.B. Ginkoflavonglykoside), Crataegus-Extrakt (z.B. oligomere Procyanidine), Buchweizen (z.B. Rutin), Sophora japonica (z.B. Rutin), Birkenblätter (z.B. Quercetinglykoside, Hyperosid und Rutin), Holunderblüten (z.B. Rutin), Lindenblüten (z.B. ätherisches Öl mit Quercetin und Farnesol), Johanniskrautöl, (z.B. Olivenölauszug), Calendula, Arnika (z.B. ölige Auszüge der Blüten mit ätherischem Öl, polare Auszüge mit Flavonoiden), Melisse (z.B. Flavone, ätherisches Öl); Immunstimulantien: Echinacea purpurea (z.B. alkoholische Auszüge, Frischpflanzensaft, Preßsaft), Eleutherokokkus senticosus; Alkaloide: Rauwolfia (z.B. Prajmalin), Immergrün (z.B.Vincamin); weitere Phytopharmaka: Aloe, Roßkastanie (z.B. Aescin), Knoblauch (z.B. Knoblauchöl), Ananas (z.B. Bromelaine), Ginseng (z.B. Ginsenoside), Mariendistelfrüchte (z.B. auf Silymarin standardisierter Extrakt), Mäusedornwurzel (z.B. Ruscogenin), Baldrian (z.B. Valepotriate, Tct. Valerianae), Kava-Kava (z.B. Kavalactone), Hopfenblüten (z.B. Hopfenbitterstoffe), Extr. Passi-florae, Enzian (z.B. ethanol. Extrakt), anthrachinonhaltige Drogenauszüge, z.B. aloinhaltiger Aloe Vera-Saft, Pollenextrakt, Algenextrakte, Süßholzwurzelextrakte, Palmenextrakt, Galphimia (z.B.Urtinktur), Mistel (z.B. wässrig-ethanol. Auszug), Phytosterole (z.B. beta-Sitosterin), Wollblumen (z.B. wäßrig-alkohol. Extrakt), Drosera (z.B. Likörweinextrakt), Sanddornfrüchte (z.B. daraus gewonnener Saft oder Sanddornöl), Eibischwurzel, Primelwurzelextrakt, Frischpflanzenextrakte aus Malve, Beinwell, Efeu, Schachtelhalm, Schafgarbe, Spitzwegerich (z.B. Preßsaft), Brennessel, Schöllkraut, Petersilie; Pflanzenextrakte aus Norolaena lobata, Tagetes lucida, Teeoma siems, Momordica charantia, und Aloe Vera Extrakte.

Bevorzugte kosmetische Wirkstoffe sind natürliche und synthetische Feuchthaltefaktoren bzw. Feuchthaltemittel, wie beispielsweise Glycerin, Polyglycerin, Sorbit, Dimethylisosorbid, Milchsäure und/oder Lactate, insbesondere Natriumlactat, Butylenglykol, Propylenglykol, Biosaccaride Gum-1, Glycine Soja, Hydroxyethylharnstoff, Ethylhexyloxyglycerin, Pyrrolidoncarbonsäure und Harnstoff, polymere Moisturizer aus der Gruppe der wasserlöslichen und/oder in Wasser quellbaren und/oder mit Hilfe von Wasser gelierbaren Polysaccharide, Hyaluronsäure, Chitosan, fucosereiche Polysaccharide, welche unter der Bezeichnung Fucogel™ 1000 von der Gesellschaft SOLABIA S.A. erhältlich sind, des weiteren Ceramide, Hautschutzmittel, Hautaufheller, Vitamine, Antioxidantien, sogenannte Antiagingmittel, antiirritative Mittel etc. Weitere bevorzugte kosmetische Wirkstoffe sind natürliche Fette und Öle, d.h. Triglyceride von natürlichen Fettsäuren, beispielsweise aufgrund ihrer rückfettenden und pflegenden Wirkung auf der Haut.

Bevorzugte kosmetische Wirkstoffe sind Antitranspirationsmittel, astringierende Mittel, desodorierende Mittel welche im International Cosmetic Ingredient Dictionary and Handbook (Volume 3, Herausgeber:T. E. Gottschlack, J. E. Bailey, The Personal Care Products Council, 13. Auflage, 2010) unter Section 3 unter den Stichworten Cosmetic Astringents, Deodorant Agents aufgeführt sind. Besonders bevorzugte Antitranspirationsmittel und astringierende Mittel sind

Aluminium-Salze wie z.B. Aluminiumchlorid AlCl3, Aluminiumsulfat Al2(SO4)3, Aluminiumchloride der empirischen Summenformel [A12(OH)mCln], wobei m+n=6, Aluminiumchlorhydrat [A12(OH)5C1] x H2O, Standard Al-Komplexe wie z.B. Locron P (Clariant), Micro-Dry (Reheis), ACH-331 (Summit), Aloxicoll PF 40 (Giulini), Aktivierte Al-Komplexe wie z.B. Reach 501 (Reheis), AACH-324 (Summit), AACH-7171 (Summit), Aloxicoll P (Giulini), Aloxicoll SD100, Aluminiumsesquichlorhydrat [A12(OH)4,5C11,5] x H2O, Standard Al-Komplexe wie z.B. Aluminum Sesquichlorohydrate (Reheis), AACH-308 (Summit), wie z.B. Aktivierte Al-Komplexe: Reach 301 (Reheis), Aluminiumdichlorhydrat [A12(OH)4C12] x H2O;

Aluminium-Zirkonium-Salze wie z.B. Aluminium/Zirkonium Trichlorhydrex Glycin [A14Zr(OH)13C13] x H2O x Gly, Standard Al/Zr-Komplexe: Rezal 33GP (Reheis), AZG-7164 (Summit), Zirkonal P3G (Giulini), Aktivierte Al/Zr-Komplexe wie z.B. Reach AZZ 902 (Reheis), AAZG-7160 (Summit), Zirkonal AP3G (Giulini), Aluminium/Zirkonium Tetrachlorhydrex Glycin [A14Zr(OH)12C14] x H2O x Gly, Standard Al/Zr-Komplexe wie z.B. Rezal 36G (Reheis), AZG-368 (Summit), Zirkonal L435G (Giulini), Aktivierte Al/Zr-Komplexe wie z.B. Reach 908 (Reheis), AAZG-7167 (Summit), Zirkonal AP4G (Giulini), Aluminium/Zirkonium Pentachlorhydrex Glycin [Al8Zr(OH)23Cl5] x H2O x Gly, Aluminium/Zirkonium Octachlorhydrex Glycin [A18Zr(OH)20C18] x H2O x Gly

Ebenso von Vorteil können aber auch Glycin-freie Aluminium/Zirkonium-Salze sein.

Besonders bevorzugte desodorierende Mittel sind z. B. Geruchsüberdecker wie die gängigen Parfümbestandteile, Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), Polyhexamethylene biguanides wie z.B. Cosmocil™ CQ (Lonza), Triclosan wie z.B. Irgasan™ DP 3000 (BASF), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden

Besonders vorteilhaft im Sinne der vorliegenden Erfindung können wasserlösliche Antioxidantien eingesetzt werden, wie beispielsweise Vitamine, z.B. Ascorbinsäure und deren Derivate. Ganz besonders vorteilhafte sind Vitamin E und dessen Derivate sowie Vitamin A und dessen Derivate.

Weitere vorteilhafte Wirkstoffe in der erfindungsgemäßen Zusammensetzung sind a-Hydroxysäure wie Glykolsäure, Milchsäure, Äpfelsäure, Weinsäure, Citronensäure und Mandelsäure, β-Hydroxysäure wie Salicylsäure sowie deren acylierten Derivate, die 2-Hydroxyalkansäure und ihre Derivate; natürliche Wirkstoffe und/oder deren Derivate, wie z.B. alpha-Liponsäure, Folsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder [beta]-Alanin sowie 8-Hexadecen-1,16-dicarbonsäure (Dioic acid, CAS-Nummer 20701-68-2; vorläufige INCI-Bezeichnung Octadecendioic acid) und/oder Licochalcon A und die Pflanzenextrakte.

Pharmazeutische bzw. therapeutische Wirkstoffe sind solche, die im Sinne des Arzneimittelgesetzes unter anderem dazu bestimmt sind, Krankheiten, Leiden, Körperschäden oder krankhafte Beschwerden zu heilen, zu lindern oder zu verhüten. Die Mittel bzw. Wirkstoffe sind für die äußere Anwendung bestimmt, wobei es sich um hautaktive Wirkstoffe aber auch um transdermale Wirkstoffe handelt kann. Sie schließen beispielsweise ein: Mittel zur Behandlung von Hautkrankheiten, wie antibakterielle Wirkstoffe, Antimykotika, antivirale Wirkstoffe, entzündungshemmende Wirkstoffe, wie Dexpanthenol, juckreizstillende Wirkstoffe, Cortison und Derivate, wie Glukokortikoide, wie Prednison, Prednisolon, Methylprednisolon, Betamethason, Dexamethason, Triamcinolon, Paramethason und Fludrocortison, Mittel zur Behandlung von Hautkrankheiten, wie der Neurodermitis, der atopischen Dermatitis etc., und Anti-Herpesmittel.

### Weitere Hilfemittel:

Die erfindungsgemäßen Zusammensetzungen können zusätzlich Zusatzstoffe enthalten, die in der Kosmetik üblich sind, wie Antioxidantien, Lichtschutzmittel und/oder andere Hilfs-und Zusatzmittel wie beispielsweise Emulgatoren, grenzflächenaktive Stoffe, Entschäumer, Verdicker, Tenside, Wirkstoffe, Feuchthaltemittel, Füllstoff, UV-Filter, Filmbildner, Lösemittel, Koaleszenzmittel, Aromastoffe, Geruchsabsorber, Parfüms, Gelbildner und/oder andere Polymerdispersionen wie beispielsweise Dispersionen auf Basis von Polyacrylaten enthalten, sensorische Additive, Weichmacher, Pigmente, Puffer, Treibmittel, Verlaufsmittel und/oder Thixotropiemittel, Geschmeidigkeitsmittel, Weichmacher, Konservierungsmittel. Die Mengen der verschiedenen Zusatzstoffe sind dem Fachmann für den einzusetzenden Bereich bekannt und liegen beispielweise im Bereich von 0,0 bis 25 gew. % bezogen auf das Gesamtgewicht der Zusammensetzung.

Die erfindungsgemäßen kosmetischen Zusammensetzungen können auch sensorische Additiv enthalten. Unter sensorische Additiv sind farblose oder weiße, mineralische oder synthetische, lamellare, sphärische oder längliche inerte Partikel oder ein nicht-partikuläres sensorisches Additiv zu verstehen, welche z.B. die sensorischen Eigenschaften der Formulierungen weiter verbessern und beispielsweise ein samtiges oder seidiges Hautgefühl hinterlassen.

Die sensorischen Additive können in der erfindungsgemäßen Zusammensetzung in einer Menge von 0,1 bis 10 Gew.-%, bezogen auf das Gesamtgewicht der Zusammensetzung, und vorzugsweise 0,1 bis 7 % enthalten sein.

Vorteilhafte partikuläre sensorische additive im Sinne der vorliegenden Erfindung sind Talkum, Glimmer (Mica), Siliciumoxid, Kaolin, Stärke und deren Derivaten (beispielweise Tapiocastärke, Distärkephosphat, Aluminium- bzw. Natrium-Stärke Octenylsuccinat und dergleichen), pyrogene Kieselsäure, Pigmente, die weder hauptsächlich UV-Filter-noch färbende Wirkung haben (wie z.B. Bornitrid etc.), Bornitrid, Calciumcarbonat, Dicalciumphosphat , Magnesiumcarbonat, Magnesiumhydrogencarbonat, Hydroxyapatite, mikrokristalline Cellulose, Pulver von synthetischen Polymeren, wie Polyamide (beispielsweise den unter der Handelsbezeichnung "Nylon^{®}" erhältlichen Polymeren), Polyethylen, Poly-β-alanine, Polytetrafluorethylen ("Teflon^{®}"), Polyacrylat, Polyurethan, Lauroyllysine, Silikonharz (beispielsweise den unter der Handelsbezeichnung "Tospearl^{®}" der Firma Kobo Products Inc. erhältlichen Polymeren), Hohlpartikel von Polyvinyliden/Acrylonitrile (Expancel^{®} der Firma Akzo Nobel) oder Hohlpartikel von Siliciumoxid (Silica Beads^{®} der Firma MAPRECOS)

Vorteilhafte nicht-partikuläre sensorische Additive können aus der Gruppe der Dimethiconole (z. B. Dow Corning 1503 Fluid der Fa. Dow Corning Ltd.), der Siliconcopolymere (z. B. Divinyldimethicone/Dimethicone Copolymer, Dow Corning HMW 2220 der Fa. Dow Corning Ltd.) oder der Siliconelastomere (z. B. Dimethicone Crosspolymer, Dow Corning 9040 Silicone Elastomer Blend der Fa. Dow Corning Ltd.) ausgewählt werden.

Gegebenfalls enthalten die erfindungsgemäßen kosmetischen Zusammensetzungen ein Konservierungsmittel. Zusammensetzungen mit hohen Wassergehalten müssen zuverlässig vor Verkeimung geschützt sein. Die wichtigsten dazu eingesetzten Konservierungsmittel sind Harnstoff-Kondensate, p-Hydroxybenzoesäureester, die Kombination von Phenoxyethanol mit Methyldibromoglutaronitril und Säurekonservierungen mit Benzoesäure, Salicylsäure und Sorbinsäure.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant^{®} (Lonza) kommerziell erhältlich ist), lodopropylbutylcarbamate (z.B. Glycacil-L^{®}Glycacil-S^{®} (Lonza), Dekaben^{®} LMB (Jan Dekker)), Parabene (p-Hydroxybenzoesäurealkylester, wie z.B. Methyl-, Ethyl-, Propylund/oder Butylparaben), Dehydroacetic Acid (Euxyl^{®} K 702 der Firma Schülke&Mayr), Phenoxyethanol, Ethanol, Benzoesäure. Vorteilhaft werden auch sogenannte Konservierungshelfer, wie beispielsweise Octoxyglycerin, Glycine, Soja, diol etc. eingesetzt.

Besondere vorteilhaft sind in der Kosmetik gebräuchliche Konservierungsmittel oder Konservierungshilfsstoffe wie Dibromdicyanobutan (2-Brom-2-brommethylglutarodinitril), Phenoxyethanol, 3-lod-2-propinylbutylcarbamat, 2-Brom-2-nitropropan-1 , 3-diol, Imidazolidinylhamstoff, 5-Chlor-2-methyl-4-isothiazolin-3-on, 2-Chloracetamid, Benzalkoniumchlorid, Benzylalkohol, Salicylsäure und Salicylate.

Die vorliegende Erfindung wird an Hand von Beispielen erläutert, wobei sie nicht als einschränkend zu verstehen sind. Alle Mengenangaben, Anteile und Prozentanteile sind, soweit nicht anders angegeben, auf das Gewicht und die Gesamtmenge bzw. auf das Gesamtgewicht der Zusammensetzungen bezogen.

### Beispiele:

Sofern nicht abweichend gekennzeichnet, beziehen sich alle Prozentangaben auf das Gewicht.

Sofern nicht abweichend vermerkt, beziehen sich alle analytischen Messungen auf Messungen bei Temperaturen von 23 °C.

Die Feststoff- bzw. Festkörpergehalte wurden entsprechend DIN EN ISO 3251 durch Erhitzen einer ausgewogenen Probe auf 105 °C bis zur Gewichtskonstanz ermittelt. Bei Gewichtskonstanz wurde durch erneutes Auswiegen der Probe der Festkörpergehalt berechnet.

NCO-Werte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 cm⁻¹) durchgeführt.

Die angegebenen Viskositäten wurden mittels Rotationsviskosimetrie nach DIN 53019 bei 23 °C mit einem Rotationsviskosimeter der Firma Anton Paar Germany GmbH, Ostfildern, DE bestimmt.

Die Bestimmung der mittleren Teilchengrößen (angegeben ist das Zahlenmittel) der Polyurethan-Dispersionen erfolgte nach Verdünnen mit entionisiertem Wasser mittels Laserkorrelations-Spektroskopie (Gerät: Malvern Zetasizer 1000, Malver Inst. Limited).

### Verwendete Substanzen und Abkürzungen:

- Diaminosulfonat:: NH₂-CH₂CH₂-NH-CH₂CH₂-SO₃Na (45 %ig in Wasser)

### Erfindungsgemäßes Beispiel: Polyurethanharnstoff-Dispersion A

1360,0 g eines Polyesters aus Adipinsäure, Hexandiol und Neopentylglykol mit einem mittleren Molekulargewicht von 1700 g/mol wurden auf 65 °C aufgeheizt. Anschließend wurden 318,5 g Isophorondiisocyanat (IPDI) zugegeben und solange bei 105 °C gerührt bis der theoretische NCO-Wert unterschritten war. Das fertige Prepolymer wurde mit 3000 g Aceton bei 50 °C gelöst und anschließend eine Lösung aus 23,4 g Isophorondiamin (IPDA), 129,6 g Diaminosulfonat und 357 g Wasser zudosiert. Die Nachrührzeit betrug 15 min. Danach wurde durch Zugabe von 2900 g Wasser dispergiert. Es folgte die Entfernung des Lösemittels durch Destillation im Vakuum und es wurde eine lagerstabile Dispersion erhalten, der Festgehalt wurde durch Zusatz von Wasser eingestellt.

| | |
|---|---|
| Feststoffgehalt: | 32 % |
| Partikelgröße (LKS): | 27 nm |
| Viskosität: | 1500 mPas |
| pH-Wert: | 7,3 |

### Erfindungsgemäßes Beispiel: Polyurethanharnstoff-Dispersion B

318,8 g eines Polyesters aus Adipinsäure, Hexandiol und Neopentylglykol mit einem mittleren Molekulargewicht von 1700 g/mol wurden auf 65 °C aufgeheizt. Anschließend wurden 70,9 g Isophorondiisocyanat zugegeben und solange bei 105 °C gerührt bis der theoretische NCO-Wert unterschritten war. Das fertige Prepolymer wurde mit 700 g Aceton bei 50°C gelöst und anschließend eine Lösung aus 3,5 g Isophorondiamin, 30,4 g Diaminosulfonat und 84 g Wasser zudosiert. Die Nachrührzeit betrug 15 min. Danach wurde durch Zugabe von 513 g Wasser dispergiert. Es folgte die Entfernung des Lösemittels durch Destillation im Vakuum und es wurde eine lagerstabile Dispersion erhalten, der Festgehalt wurde durch Zusatz von Wasser eingestellt.

| | |
|---|---|
| Feststoffgehalt: | 30 % |
| Partikelgröße (LKS): | 32 nm |
| Viskosität: | 1000 mPas |
| pH-Wert: | 7,2 |

### Erfindungsgemäßes Beispiel: Polyurethanharnstoff-Dispersion C

319 g eines Polyesters aus Adipinsäure, Hexandiol und Neopentylglykol mit einem mittleren Molekulargewicht von 1700 g/mol wurden auf 65 °C aufgeheizt. Anschließend wurden 79,2 g Isophorondiisocyanat zugegeben und solange bei 105 °C gerührt bis der theoretische NCO-Wert unterschritten war. Das fertige Prepolymer wurde mit 1100 g Aceton bei 50°C gelöst und anschließend eine Lösung aus 8,0 g Isophorondiamin, 30,4 g Diaminosulfonat und 84 g Wasser zudosiert. Die Nachrührzeit betrug 15 min. Danach wurde durch Zugabe von 540 g Wasser dispergiert. Es folgte die Entfernung des Lösemittels durch Destillation im Vakuum und es wurde eine lagerstabile Dispersion erhalten, der Festgehalt wurde durch Zusatz von Wasser eingestellt.

| | |
|---|---|
| Feststoffgehalt: | 28 % |
| Partikelgröße (LKS): | 35 nm |
| Viskosität: | 760 mPas |
| pH-Wert: | 7,7 |

### Beispielformulierungen für haut- und Haarkosmetische Formulierungen:

Alle Mengenangaben, Anteile und Prozentanteile sind, soweit nicht anders angegeben, auf das Gewicht und die Gesamtmenge bzw. auf das Gesamtgewicht der Zusammensetzung bezogen.

Bei dem in den folgendeTabellen genannten erfindungsgemäßen Polyurethanharnstoff handelt es sich um Polyurethanharnstoffdispersion A

### 1. Skin Care Formulierungen

### 1.1 O/W-Emulsion

| | 1 | 2 | 3 | 4 | 5 | 6 | 7 | 8 | 9 | 10 |
|---|---|---|---|---|---|---|---|---|---|---|
| Erfindungsgemäßer Polyurethanharnstoff (bezogen auf Feststoff) | 5,0 | 7,0 | 8,0 | 10 | 15, 0 | 2,0 | 10, 0 | 6,0 | 5,0 | 3,0 |
| Glyceryl Stearate Citrate | 2,0 | | | | | | | | | |
| Glyceryl Stearate (Selbtsemulgierend) | | 2,0 | | | | | | | | |
| Polyglyceryl-3 Methylglucose Distearate | | | 2,5 | 2,0 | | | | | | |
| Sorbitan Stearate | | | | 1,0 | | | | | | |
| PEG-40 Stearate | | | | | 0,5 | | | | | |
| Glyceryl Stearate | | | | | 2,5 | 0,5 | 2,0 | | 2,0 | |
| PEG-100 Stearate | | | | | | | 2,0 | | | |
| Sodium Stearoyl Glutamate | | | | | | | | 0,5 | 0,2 | |
| Distearyldiammonium chloride | | | | | | | | | | 1,0 |
| Stearic acid | | 1,0 | | | | | | | | |
| Behenyl alcohol | | | | | | 1,0 | | | | |
| Cetyl alcohol | | | | | | | | | | 2,5 |
| Cetearyl alcohol | | 2,0 | | | 5,0 | 10,0 | 2,0 | | | |
| Myristyl alcohol | | 2,0 | | | | | | | | |
| Stearyl alcohol | 1,0 | | 1,0 | | | | | | 3,0 | 1,0 |
| Acrylates/C10-30 Alkyl Acrylate crosspolymer¹ | 0,1 | | | | | | 0,8 | | 0,3 | |
| Ammonium Acryloyldimethyltaurate / VP Copolymer² | | 0,5 | | | | | | | | |
| Acrylic Acid/VP Crosspolymer³ | | | | 0,6 | | | | 0,2 | | |
| Carbomer | | | 0,8⁴ | | 0,3⁵ | | | | 0,5⁶ | |
| Dimethylpolysiloxane | | | | | | | | | | |
| Dicaprylyl Ether | 1,0 | 3,0 | | | | | | | | 1,0 |
| Myristyl Myristate | 3,0 | | | | | | | 1,0 | | |
| Octyldodecanol | 1,0 | | | 4,0 | 5,0 | 3,0 | | | 4,0 | |
| Butylene Glycol Dicaprylate/Dicaprate | | 2,0 | | | 3,0 | | | | | |
| C12-15 Alkyl Benzoate | | 3,0 | | | 3,0 | | 5,0 | 1,0 | | |
| Isohexadecane | | | 2,0 | | | | 3,0 | | | |
| Caprylic/Capric Triglycerid | | | | 2,0 | | | | 2,0 | | |
| Cyclomethicone | 4,0 | | 2,0 | | | 2,0 | | | 1,0 | |
| Dimethicone | 2,0 | | 1,0 | | | 2,0 | 5,0 | | 1,0 | |
| Mineral Oil | | | | | | 5,0 | | | | 2,5 |
| Hydrietes Polyisobutene | | | | | | 2,0 | | | | |
| Phenethylbenzoate | | | | | | | | 5,0 | | |
| Isodecyl Neopentanoate | | | | | | | | | 2,0 | |
| Oenothera Biennis | | | | | | | | | | 2,0 |
| Shea Butter | | | | | | | | | | 2,0 |
| Butylenglycol | | | | 5,0 | | | | | | |
| Glycerin | 10 | 7,5 | 5,0 | | 3,0 | | 10 | 8,0 | 5,0 | 5,0 |
| Ethanol | 3,0 | | | | | | | | 4,0 | |
| Tapioca starch⁷ | 1,0 | | 1,0 | | | | | | | |
| Distarch Phosphate | | 2,0 | | | | | | | | |
| Aluminium Starch⁸ Octenylsuccinate | | | | | | | | 2,0 | | |
| Natrium starch Octenylsucccinate⁹ | | | | | 2,0 | | | | | |
| Butyl Methoxydibenzoylmethane | | | | | | | | 4 | | |
| Octocrylene | | | | | | | | 5 | | |
| Phenylbenzimidazole sulfonic acid | | | 2 | | | | | 2 | | |
| Ethylhexyl Methoxycinnamate | | | 5 | | | | | | | |
| Trisodium EDTA | | | 1 | | | | | 1 | | |
| Wirkstoffe | q. s. | q. s. | q. s. | q. s. | q. s. | q. s. | q. s. | q. s. | q. s. | q. s. |
| Neutralisationsmittel | q. s. | q. s. | q. s. | q. s. | q. s. | q. s. | q. s. | q. s. | q. s. | q. s. |
| Farbstoffe | q. s. | q. s. | q. s. | q. s. | q. s. | q. s. | q. s. | q. s. | q. s. | q. s. |
| Parfüm | q. s. | q. s. | q. s. | q. s. | q. s. | q. s. | q. s. | q. s. | q. s. | q. s. |
| Konservierungsmittel | q. s. | q. s. | q. s. | q. s. | q. s. | q. s. | q. s. | q. s. | q. s. | q. s. |
| Aqua | Ad. 100 | Ad. 100 | Ad. 100 | Ad. 100 | Ad. 100 | Ad. 100 | Ad. 100 | Ad. 100 | Ad. 100 | Ad. 100 |

| | | | | | | | | | | |
|---|---|---|---|---|---|---|---|---|---|---|
| ¹Pemulen TR-1, Lubrizol ²Aristoflex AVC, Clariant ³UltraThix P-100,Ashland ⁴Carbopol 980, Lubrizol ⁵Carbopol Ultrez 10, Lubrizol ⁶Carbopol 981, Lubrizol ⁷Tapioca Pure, AkzoNobel ⁸Dry Flo-PC, AkzoNobel ⁹Cleargum CO 01, Roquette | | | | | | | | | | |

### 1.2 W/O-Emulsion

| | 1 | 2 | 3 | 4 | 5 |
|---|---|---|---|---|---|
| Erfindungsgemäßer Polyurethanharnstoff (bezogen auf Feststoff) | 6,0 | 2,5 | 8,0 | 5,0 | 10,0 |
| Polygylceryl-3 Diisostearate | 1,0 | | | | |
| Polyglyceryl-2 Dipolyhydroxystearate | 3,0 | 3,0 | | | |
| PEG-40 Sorbitan Perisostearate | | 3,0 | | | |
| Triglycerindüsostearate | | | 0,5 | | |
| Diglycerindipolyhydroxytsearate | | | 1,5 | | |
| PEG-30 Dipolyhydroxystearate | | | | 0,25 | |
| PEG-22 Dodecyl Glycol Copolymer | | | | | 5,0 |
| PEG-45 Dodecyl Glycol Polymer | | | | | 1,0 |
| Lanolin Alcohol | 1,0 | | | 0,3 | 0,5 |
| Behenyl Alcohol | | | 0,5 | | |
| Caprylic/Capric Triglyceride | 15,0 | 15,0 | | | |
| Mineral oil | 10,0 | 8,0 | 10,0 | 8,0 | 10,0 |
| Cera Microcrystallina | 5,0 | | 1,0 | | |
| Dicaprylylcarbonate | 1,0 | | | | |
| Isopropylstearate | | | | | 8,0 |
| Isopropylpalmitate | 1,0 | | | | |
| Rizinus oil | 1,0 | | | | |
| Vaseline | | | 6,0 | 5,0 | |
| Octyldodecanol | | | 1,0 | 3,0 | |
| Hydrogenated Cocoglyceride | | | | 2,0 | |
| Oenothera Biennis | | | | | 0,5 |
| Aluminium stearate | | | 0,3 | 0,6 | 0,5 |
| Magnesiumsulfate | 0,5 | 1,0 | 0,5 | 0,5 | 0,5 |
| Sodium citrate | 0,5 | 0,3 | 0,05 | | 0,2 |
| Sodium chloride | | | | 10,0 | |
| Citric acid | | | 0,1 | 0,2 | 0,2 |
| Potassium sorbate | | | 0,15 | | 0,4 |
| Glycerin | 3,0 | 8,0 | 5,0 | 3,0 | |
| Talc | | | | 0,5 | |
| Ethanol | | | 2,0 | | |
| Wirkstoffe | q. s. | q. s. | q. s. | q. s. | q. s. |
| Neutralisationsmittel | q. s. | q. s. | q. s. | q. s. | q. s. |
| Farbstoffe | q. s. | q. s. | q. s. | q. s. | q. s. |
| Parfüm | q. s. | q. s. | q. s. | q. s. | q. s. |
| Konservierungsmittel | q. s. | q. s. | q. s. | q. s. | q. s. |
| Aqua | Ad. 100 | Ad. 100 | Ad. 100 | Ad. 100 | Ad. 100 |

### 1.3 W/Si-Emulsion

| | 1 | 2 | 3 |
|---|---|---|---|
| Erfindungsgemäßer Polyurethanharnstoff (bezogen auf Feststoff) | 5,0 | 10,0 | 2,5 |
| Cetyl dimethicone Copolyol | 2,0 | | |
| Cetyl PEG/PPG-10/1 Dimethicone | | 3,0 | 3,0 |
| Cyclomethicone | 15,0 | 25,0 | 25,0 |
| Dimethicone | 15,0 | 5,0 | 5,0 |
| Phenyltrimethicon | 1,0 | | |
| Hydrogenated Polyisobutene | | 2,0 | 2,0 |
| Dimethiconol | | 1,0 | 1,0 |
| Xanthan gum¹⁰ | 0,1 | | |
| Glycerin | 5,0 | 2,0 | 2,0 |
| Magnesium sulfate | 1,0 | | |
| Sodium chloride | | 0,7 | 0,7 |
| Citric acid | | 0,3 | 0,3 |
| Sodium citrate | | 0,9 | 0,9 |
| Potassium sorbate | | 0,3 | 0,3 |
| Wirkstoffe | q. s. | q. s. | q. s. |
| Neutralisationsmittel | q.s. | q.s. | q.s. |
| Farbstoffe | q. s. | q. s. | q. s. |
| Parfüm | q. s. | q. s. | q. s. |
| Konservierungsmittel | q. s. | q. s. | q. s. |
| Aqua | Ad. 100 | Ad. 100 | Ad. 100 |

| | | | |
|---|---|---|---|
| ¹⁰ Keltrol CG-T, CP Kelco | | | |

### 1.4 Hydrodispersion

| | 1 | 2 | 3 | 4 | 5 |
|---|---|---|---|---|---|
| Erfindungsgemäßer Polyurethanharnstoff (bezogen auf Feststoff) | 5,0 | 10,0 | 2,5 | 5,0 | 8,0 |
| Cetearyl Alkohol + PEG-40 Rizinusöl - Natrium Cetearyl Sulfat | 2,5 | | | | |
| Sorbitan Stearate | 1,0 | | | | |
| Ceteareth-20 | | | | 0,5 | |
| Ammonium Acryloyldimethyltaurate /VP Copolymer¹¹ | | | | | 1,0 |
| Acrylates/C10-30 Alkyl Acrylate crosspolymer¹² | 0,8 | 0,3 | 0,3 | 1,0 | |
| Xanthan gum¹³ | | 0,5 | 0,5 | | |
| Octyldodecanol | 2,0 | 2,0 | 2,0 | | 2,0 |
| Caprylic/Capric Triglycerid | 3,0 | 3,0 | 3,0 | | 2,0 |
| Cyclomethicone | 4,0 | | | | 2,0 |
| Isodecyl Neopentanoate | | | | 3,0 | |
| Dimethicone | | | | 2,0 | |
| Dicaprylylcarbonat | | | | | 2,0 |
| Sodium Starch Octenylsuccinat | 1,5 | | | | |
| Tapioka Starch | 3.0 | | | | 1,0 |
| Alcohol | 3.0 | | | | |
| Glycerin | 5,0 | 2,0 | 2,0 | 5,0 | 2,0 |
| Ethylhexyl Methoxycinnamate | | 8,0 | 8,0 | | |
| Octocrylene | | 5,0 | 5,0 | | |
| Phenylbenzimidazol Sulfonsäure | | 2,0 | 2,0 | | |
| Ethanol | | | | 5,0 | |
| Sodium starch Octenylsucccinate¹⁴ | | | | 0,5 | |
| Wirkstoffe | q. s. | q. s. | q. s. | q. s. | q. s. |
| Neutralisationsmittel | q. s. | q. s. | q. s. | q. s. | q. s. |
| Farbstoffe | q. s. | q. s. | q. s. | q. s. | q. s. |
| Parfüm | q. s. | q. s. | q. s. | q. s. | q. s. |
| Konservierungsmittel | q. s. | q. s. | q. s. | q. s. | q. s. |
| Aqua | Ad. 100 | Ad. 100 | Ad. 100 | Ad. 100 | Ad. 100 |

| | | | | | |
|---|---|---|---|---|---|
| ¹¹Aristoflex AVC, Clariant ¹² Pemulen TR-1, Lubrizol ¹³ Keltrol CG-T, CP Kelco ¹⁴ Cleargum CO 01, Roquette | | | | | |

### 2. Sun Care

### 2.1 O/W-Emulsion

| | 1 | 2 | 3 | 4 | 5 | 6 | 7 | 8 | 9 | 10 | 11 | 12 | 13 |
|---|---|---|---|---|---|---|---|---|---|---|---|---|---|
| Erfindungsgemäßer Polyurethanharnstoff (bezogen auf Feststoff) | 7,5 | 5,0 | 3,0 | 2,0 | 15,0 | 3,0 | 10,0 | 4,0 | 5,0 | 2,0 | 5,0 | 7,5 | 7,50 |
| VP/Eicosene Copolymer | 0,5 | | | | 2,0 | | | | | | | | |
| PVP/Hexadeca ne Copolymer | | | | 0,5 | | | | | | | | | |
| Diglyglycol/CHDM/Iso phtalates/SIP Copolymer | | | 3,0 | | | | | | | | | | |
| Sorbitan laurate (and) Polyglyceryl-10 laurate | | | | | | | | | | | | | 2,5 |
| Glyceryl Stearate Citrate | 2,0 | | | | | | | | | | | | |
| Glyceryl Stearate (Selbtsemulgierend) | | 2,0 | | | | | | | | | 2,0 | | |
| Polygyceryl-2 Dipolyhyhroxystearat | | | | | | | | | | | | 0,25 | |
| Polyglyceryl-3 Methylglucose Distearate | | | 2,5 | 2,0 | | | | | | | | | |
| Sorbitan stearate | | | | 1,0 | | | | | | | | | |
| Glyceryl Isostearate | | | | | | | | | | | | 3,5 | |
| Isoceteth-20 | | | | | | | | | | | 0,5 | 2,0 | |
| Ceteareth-12 | | | | | | | | | | | 5,0 | | |
| PEG-40 Stearate | | | | | 0,5 | | | | | | | | |
| Glyceryl Stearate | | | | | 2,5 | 0,5 | 2,0 | | 2,0 | | | | |
| PEG-100 Stearate | | | | | | | 2,0 | | | | | | |
| Sodium Stearoyl Glutamate | | | | | | | | 0,5 | 0,2 | | | | |
| Distearyldiammonium Chloride | | | | | | | | | | 1,0 | | | |
| Stearic acid | | 1,0 | | | | | | | | | | | |
| Behenyl Alcohol | | | | | | 1,0 | | | | | | | |
| Cetyl Alcohol | | | | | | | | | | 2,5 | 1,0 | | |
| Cetearyl Alcohol | | 2,0 | | | 5,0 | 10,0 | 2,0 | | | | | | |
| Myristyl Alcohol | | 2,0 | | | | | | | | | | | |
| Stearyl Alcohol | 1,0 | | 1,0 | | | | | | 3,0 | 1,0 | | | |
| Ethylhexyl stearate | | | | | | | | | | | | | 7,0 |
| Acrylates/C10-30 Alkyl Acrylate crosspolymer¹⁵ | 0,1 | | | | | | 0,8 | | 0,3 | | | | 0,2 |
| Ammonium Acryloyldimethyltaurate /VP Copolymer¹⁶ | | 0,5 | | | | | | | | | | | |
| Acrylic Acid/VP Cross-polymer¹⁷ | | | | 0,6 | | | | 0,2 | | | | | |
| Carbomer | | | 0,8 18 | | 0,3¹⁹ | | | | 0,5 20 | | | | |
| Xanthan gum | | | | | | | | | | | | | 0,15 |
| Dimethylpolysiloxan | | | | 5 | | | | | | | | | |
| Dicaprylyl Ether | 1,0 | 3,0 | | | | | | | | 1,0 | | | |
| Myristyl Myristate | 3,0 | | | | | | | 1,0 | | | | | |
| Octyldodecanol | 1,0 | | | 4,0 | 5,0 | 3,0 | | | 4,0 | | 3,0 | | |
| Butylene Glycol Dicaprylate/Dicaprate | | 2 | | 2,0 | 3,0 | | | 2 | | 7,0 | | | |
| C12-15 alkyl Benzoate | | 2 | | | 3,0 | 1 | 5,0 | 2 | 5,0 | | | 5,0 | 6,0 |
| Isohexadecan | | | 2,0 | | | | 3,0 | | | | | | 1,5 |
| Caprylic/Capric Triglycerid | | | | 2,0 | | | | 2,0 | | | | | |
| Cyclomethicone | 4,0 | | 2,0 | | | 2,0 | | | 1,0 | | | | |
| Dimethicone | 2,0 | | 1,0 | | | 2,0 | 5,0 | | 1,0 | | | | |
| Mineral Oil | | | | | | 5,0 | | | | 2,5 | | | |
| Hydrietes Polyisobutene | | | | | | 2,0 | | | | | | | |
| Phenethylbenzoate | | | | | | | | 5,0 | | | | | |
| Isodecyl Neopentanoate | | | | | | | | | 2,0 | | | | |
| Oenothera Biennis | | | | | | | | | | 2,0 | | | |
| Shea Butter | | | | | | | | | | 2,0 | | | |
| Butylenglycol | | | | 5,0 | | | | | | | | | |
| Glycerin | 10 | 7,5 | 5,0 | | 3,0 | | 10 | 8,0 | 5,0 | 5,0 | 5,0 | 7,0 | 5,0 |
| Ethylhexyl-glycerin | 0,5 | | | | | 0,5 | | | | | | | |
| Ethanol | 3,0 | | | | | | | | 4,0 | | | | |
| Tapioca starch²¹ | 1,0 | | 1,0 | | | | | | | | | | |
| Distarch Phosphate | | 2,0 | | | | | | | | | | | |
| Aluminium starch ²² Octenylsuccinat | | | | | | | | 2,0 | | | | | |
| Natrium starch Octenylsucccinate²³ | | | | | 2,0 | | | | | | | | |
| Bisethylhexyloxy phenol Methoxyphenyl Triazine | | | 1 | | | | | | 2,0 | 3,0 | | | |
| Ethylhexyl Triazone | | | | 2,0 | | | | | 2,0 | | | | |
| Butyl Methoxydibenzoylmethane | 3 | 3 | | | 2,5 | 4 | | 2 | 3,0 | 1,0 | 2,0 | 3,0 | 2,0 |
| Ethylhexyl Methoxycinnamate | 4 | 4 | 3 | | 5,0 | | 5,0 | 5 | 5,0 | | 5,0 | | 7,0 |
| Octocrylene | | | | | 4,0 | 5 | | | | | | 4,0 | |
| Phenylbenzimidazole Sulfonic Acid | | | | 1,0 | 2,0 | 2 | 2,0 | | | | | | 4,0 |
| Titanium Dioxide + Trimethoxycaprylylsilane | | | | | | 1 | | | | | | | |
| Terephthalidene dicamphor sulfonic acid | | | | | | | | | | | | 3,0 | |
| Diethylamino Hydrobenzoyl Hexylbenzoat | | | | | | | 1,0 | | | | | | |
| Phenylen-1,4bis-(2-benzimidazol)-3,3,5,5'-tetrasulfonic acid | | | | 1,0 | | | 2,0 | | | | | | |
| Polysilicon-15 | | | | | | | | | | 3,0 | | | |
| Benzophenone-3 | | | | | | | | | | | 2,0 | | |
| Titanium Dioxide | | | | 2,0 | | | | | | | | | |
| Trisodium EDTA | 1 | 1 | 1 | | | | 1 | 1 | 1 | 1 | 1 | | 1 |
| Wirkstoffe | q. s. | q. s. | q. s. | q. s. | q. s. | q. s. | q. s. | q. s. | q. s. | q. s. | q. s. | q. s. | q. s. |
| Neutralisationsmittel | q. s. | q. s. | q. s. | q. s. | q. s. | q. s. | q. s. | q. s. | q. s. | q. s. | q. s. | q. s. | q. s. |
| Farbstoffe | q. s. | q. s. | q. s. | q. s. | q. s. | q. s. | q. s. | q. s. | q. s. | q. s. | q. s. | q. s. | q. s. |
| Parfüm | q. s. | q. s. | q. s. | q. s. | q. s. | q. s. | q. s. | q. s. | q. s. | q. s. | q. s. | q. s. | q. s. |
| Konservierungsmittel | q. s. | q. s. | q. s. | q. s. | q. s. | q. s. | q. s. | q. s. | q. s. | q. s. | q. s. | q. s. | q. s. |
| Aqua | Ad. 100 | Ad. 100 | Ad. 100 | Ad. 100 | Ad. 100 | Ad. 100 | Ad. 100 | Ad. 100 | Ad. 100 | Ad. 100 | Ad. 100 | Ad. 100 | Ad. 100 |

| | | | | | | | | | | | | | |
|---|---|---|---|---|---|---|---|---|---|---|---|---|---|
| ¹⁵Pemulen TR-1, Lubrizol ¹⁶Anstoflex AVC, Clanant ¹⁷UltraThix P-100, Ashland ¹⁸Carbopol 980, Lubrizol ¹⁹Carbopol Ultrez 10, Lubrizol ²⁰Carbopol 981, Lubrizol ²¹ Tapioca Pure, AkzoNobel ²² Dry Flo-PC, AkzoNobel ²³ Cleargum CO 01, Roquette | | | | | | | | | | | | | |

### 2.2 W/O-Emulsion

| | 1 | 2 | 3 | 4 | 5 | 6 | 7 |
|---|---|---|---|---|---|---|---|
| Erfindungsgemäßer Polyurethanharnstoff (bezogen auf Feststoff) | 2,0 | 5,0 | 8,0 | 7,5 | 5,0 | 2,0 | 10,0 |
| VP/Eicosene Copolymer | 1,0 | | | | | | |
| PVP/Hexadecane Copolymer | | 0,5 | | | | | |
| Polygylceryl-3 Diisostearate | 1,0 | | | 1,0 | | | |
| Polyglyceryl-2 Dipolyhydroxystearate | 3,0 | 3,0 | | 3,0 | | | |
| PEG-40 Sorbitan Perisostearate | | 3,0 | | | | | |
| Triglycerindüsostearate | | | 0,5 | | 0,5 | | |
| Diglycerindipolyhydroxytsearate | | | 1,5 | | 1,5 | | |
| PEG-30 Dipolyhydroxystearate | | | | | | 0,25 | |
| PEG-22 Dodecyl Glycol Copolymer | | | | | | | 5,0 |
| PEG-45 Dodecyl Glycol Polymer | | | | | | | 1,0 |
| Lanolin Alcohol | 1,0 | | | 1,0 | | 0,3 | 0,5 |
| Behenyl Alcohol | | | 0,5 | | 0,5 | | |
| Caprylic/Capric Triglyceride | 15,0 | 15,0 | | 15,0 | | | |
| Mineral oil | 10,0 | 8,0 | 10,0 | 10,0 | 10,0 | 8,0 | 10,0 |
| Cera Microcrystallina | 5,0 | | 1,0 | 5,0 | 1,0 | | |
| Dicaprylylcarbonate | 1,0 | | | 1,0 | | | |
| Isopropylstearate | | | | | | | 8,0 |
| Isopropylpalinitate | 1,0 | | | 1,0 | | | |
| Rizinus oil | 1,0 | | | 1,0 | | | |
| Vaseline | | | 6,0 | | 6,0 | 5,0 | |
| Octyldodecanol | | | 1,0 | | 1,0 | 3,0 | |
| Hydrierte Kokoglyceride | | | | | | 2,0 | |
| Oenothera Biennis | | | | | | | 0,5 |
| Butylene Glycol Dicaprylate/Dicaprate | | | 2 | | 2 | | |
| C12-15 Alkyl Benzoate | | 4 | 2 | | 2 | 1 | 4 |
| Aluminiumstearate | | | 0,3 | | 0,3 | 0,6 | 0,5 |
| Magnesiumsulfate | 0,5 | 1,0 | 0,5 | 0,5 | 0,5 | 0,5 | 0,5 |
| Sodium citrate | 0,5 | 0,3 | 0,05 | 0,5 | 0,05 | | 0,2 |
| Sodium chloride | | | | | | 10,0 | |
| Citric acid | | | 0,1 | | 0,1 | 0,2 | 0,2 |
| Potassium sorbate | | | 0,15 | | 0,15 | | 0,4 |
| Ethylhexylglycerin | 0,5 | | | 0,5 | | | |
| Glycerin | 3,0 | 8,0 | 5,0 | 3,0 | 5,0 | 3,0 | |
| Trisodium EDTA | | 1 | 1 | | 1 | | |
| Ethylhexyl Triazone | | | | | | | |
| Butyl Methoxydibenzoylmethane | | 2,5 | | | | 4 | 2 |
| Ethylhexyl Methoxycinnamate | | | 3 | | 3 | | 5 |
| Octocrylene | 3 | | | 3 | | 5 | |
| Phenylbenzimidazole Sulfonic Acid | | 4 | | | | 2 | |
| Titanium Dioxide | 1 | | | 1 | | 1 | |
| Talc | | | | | | 0,5 | |
| Ethanol | | | 2,0 | | 2,0 | | |
| Trisodium EDTA | | 1 | 1 | | 1 | | |
| Wirkstoffe | q.s. | q.s. | q.s. | q.s. | q.s. | q.s. | q.s. |
| Neutralisationsmittel | q.s. | q.s. | q.s. | q.s. | q.s. | q.s. | q.s. |
| Farbstoffe | q.s. | q.s. | q.s. | q.s. | q.s. | q.s. | q.s. |
| Parfüm | q.s. | q.s. | q.s. | q.s. | q.s. | q.s. | q.s. |
| Konservierungsmittel | q.s. | q.s. | q.s. | q.s. | q.s. | q.s. | q.s. |
| Aqua | Ad. 100 | Ad. 100 | Ad. 100 | Ad. 100 | Ad. 100 | Ad. 100 | Ad. 100 |

### 2.3 W/Si-Emulsion

| | 1 | 2 | 3 |
|---|---|---|---|
| Erfindungsgemäßer Polyurethanharnstoff (bezogen auf Feststoff) | 5,0 | 2,5 | 10,0 |
| Cetyl dimethicone Copolyol | 2,0 | 2,0 | |
| Cetyl PEG/PPG-10/1 Dimethicone | | | 3,0 |
| Cyclomethicon | 15,0 | 15,0 | 25,0 |
| Dimethicone | 15,0 | 15,0 | 5,0 |
| Phenyltrimethicon | 1,0 | 1,0 | |
| Hydrierte Polyisobuten | | | 2,0 |
| Dimethiconol | | | 1,0 |
| Xanthan gum²⁴ | 0,1 | 0,1 | |
| Ethylhexylglycerin | | | 0,5 |
| Glycerin | 5,0 | 5,0 | 2,0 |
| Magnesium sulfate | 1,0 | 1,0 | |
| Natrium chloride | | | 0,7 |
| Citric acid | | | 0,3 |
| Sodium citrat | | | 0,9 |
| Potassium sorbate | | | 0,3 |
| Trisodium EDTA | | | 1 |
| Ethylhexyl Triazone | 2 | 2 | |
| Butyl Methoxydibenzoylmethane | 3 | 3 | |
| Ethylhexyl Methoxycinnamate | | | 2 |
| Titanium Dioxide | 0,5 | 0,5 | 2 |
| Wirkstoffe | q.s. | q.s. | q.s. |
| Neutralisationsmittel | q.s. | q.s. | q.s. |
| Farbstoffe | q.s. | q.s. | q.s. |
| Parfüm | q.s. | q.s. | q.s. |
| Konservierungsmittel | q.s. | q.s. | q.s. |
| Aqua | Ad. 100 | Ad. 100 | Ad. 100 |

| | | | |
|---|---|---|---|
| ²⁴ Keltrol CG-T, CP Kelco | | | |

### 2.4 Hydrodispersion

| | 1 | 2 | 3 | 4 | 5 | 6 |
|---|---|---|---|---|---|---|
| Erfindungsgemäßer Polyurethanharnstoff (bezogen auf Feststoff) | 2,5 | 7,5 | 10,0 | 5,0 | 5,0 | 8,0 |
| VP/Eicosene Copolymer | 0,5 | | | | | |
| PVP/Hexadecane Copolymer | | | | 0,5 | | |
| Cetearyl Alkohol + PEG-40 Rizi- | 2,5 | 2,5 | | | | |
| nusöl - Natrium Cetearyl Sulfat | | | | | | |
| Sorbitan Stearat | 1,0 | 1,0 | | | | |
| Ceteareth-20 | | | | 0,5 | 0,5 | |
| Ammonium Acryloyldimethyltaurate /VP Copolymer 21 | | | | | | 1,0 |
| Acrylates/C10-30 Alkyl Acrylate crosspolymer²⁶ | 0,8 | 0,8 | 0,3 | 1,0 | 1,0 | |
| Xanthan gum²⁷ | | | 0,5 | | | |
| Octyldodecanol | 2,0 | 2,0 | 2,0 | | | 2,0 |
| Caprylic/Capric Triglycerid | 3,0 | 3,0 | 3,0 | | | 2,0 |
| Cyclomethicon | 4,0 | 4,0 | | | | 2,0 |
| Isodecyl Neopentanoate | | | | 3,0 | 3,0 | |
| Dimethicone | | | | 2,0 | 2,0 | |
| Dicaprylylcarbonat | | | | | | 2,0 |
| Butylene Glycol Dicaprylate/Dicaprate | 2,0 | 2,0 | | 5,0 | 5,0 | |
| C12-15 Alkyl Benzoate | 2,0 | 2,0 | | | | 5,0 |
| Natrium Starch Octenylsuccinat | 1,5 | 1,5 | | | | |
| Tapioka Starch | 3.0 | 3.0 | | | | 1,0 |
| Ethanol | 3.0 | 3.0 | | | | |
| Glycerin | 5,0 | 5,0 | 2,0 | 5,0 | 5,0 | 2,0 |
| Ethylhexyl Methoxycinnamat | 3,0 | 3,0 | 8,0 | | | |
| Octocrylen | | | 5,0 | | | |
| Phenylbenzimidazol Sulfonsäure | | | 2,0 | | | |
| Butyl Methoxydibenzoylmethane | 2,0 | 2,0 | | 3,0 | 3,0 | 3,0 |
| Ethylhexyl Triazone | | | | 2,0 | 2,0 | 2,0 |
| Bis-Ethylhexyloxyphenol Methoxyphenyl Triazine | | | | | | 2,0 |
| Titanium Dioxide | | | | 0,5 | 0,5 | |
| Trisodium EDTA | 1,0 | 1,0 | | 1,0 | 1,0 | 1,0 |
| Ethanol | | | | 5,0 | 5,0 | |
| Natrium starch Octenylsucccinate²⁸ | | | | 0,5 | 0,5 | |
| Wirkstoffe | q. s. | q. s. | q. s. | q. s. | q. s. | q. s. |
| Neutralisationsmittel | q. s. | q. s. | q. s. | q. s. | q. s. | q. s. |
| Farbstoffe | q. s. | q. s. | q. s. | q. s. | q. s. | q. s. |
| Parfüm | q. s. | q. s. | q. s. | q. s. | q. s. | q. s. |
| Konservierungsmittel | q. s. | q. s. | q. s. | q. s. | q. s. | q. s. |
| Aqua | Ad. 100 | Ad. 100 | Ad. 100 | Ad. 100 | Ad. 100 | Ad. 100 |

| | | | | | | |
|---|---|---|---|---|---|---|
| ²⁵Aristoflex AVC, Clariant ²⁶Pemulen TR-1, Lubrizol ²⁷Keltrol CG-T, CP Kelco ²⁸Cleargum CO 01, Roquette | | | | | | |

### 3. Dekorative Kosmetik

### 3.1 Mascara

| | 1 | 2 | 3 | 4 | 5 |
|---|---|---|---|---|---|
| Isododecane | 20,0 0 | 20,0 0 | 20,0 0 | | |
| D5 Cyclomethicone | 5,00 | 5,00 | 5,00 | | |
| Simethicone | | | | | 0,1 |
| Carnauba Wax | 6,00 | 6,00 | 6,00 | 6,4 | 3,5 |
| Beewax | | | | | 7,4 |
| Ceresin Wax SP252 | 3,00 | 3,00 | 3,00 | | |
| Paraffin Wax 130/135 | 3,50 | 3,50 | 3,50 | | |
| Polyethylene | 2,50 | 2,50 | 2,50 | | |
| Rice Bran Wax | | | | 9,6 | |
| Candelilla Wax | | | | 3,2 | |
| Acacia Gum | | | | 2,7 | |
| Trimethyl Siloxysilicate | 0,75 | 0,75 | 0,75 | | |
| Dimethicone 200/200 | 10,0 0 | 10,0 0 | 10,0 0 | | |
| PVP/Eicosene Copolymer | 7,5 | 7,5 | 7,5 | | |
| Erfindungsgemäßer Polyurethanharnstoff (bezogen auf Feststoff) | 15,0 | 5,0 | 7,0 | 18,0 | 10,0 |
| Acrylates Copolymer²⁹ | | 10,0 | | | |
| PPG-17/IPDI/DMPA Copolymer³⁰ | | | 7,0 | | |
| Nylon-12 | 2,00 | 2,00 | 2,00 | | |
| Silica | 2,00 | 2,00 | 2,00 | | |
| Acrylates/C10-30 Alkyl Acrylate crosspolymer³¹ | | | | | 0,8 |
| PEG-200 Glyceryl Stearate | | | | | 4,0 |
| Potassium Cetyl Phosphate | | | | 1,3 | |
| Stearic Acid | 1,00 | 1,00 | 1,00 | | |
| Steareth-2 | | | | 1,7 | |
| Steareth-20 | | | | 3,6 | |
| Cetyl Alcohol | | | | 1,6 | |
| Bentone Gel in Isododecane | 15,0 0 | 15,0 0 | 15,0 0 | | |
| Hydroxyethylcellulose | | | | 0,6 | |
| Konservierungsmittel | q. s. | q. s. | q. s. | q. s. | q. s. |
| Black Iron Oxide LC989 EM | 10,0 0 | 10,0 0 | 10,0 0 | 12,0 | 8,0 |
| Wirkstoffe | q. s. | q. s. | q. s. | q. s. | q. s. |
| White Beeswax | 1,75 | 1,75 | 1,75 | | |
| Deionized Water | Ad. 100 | Ad. 100 | Ad. 100 | Ad. 100 | Ad. 100 |
| Magnesium Aluminium Silicate | 0,50 | 0,50 | 0,50 | | |
| Triethanolamine 99 % | 0,90 | 0,90 | 0,90 | | |
| Ethanol | | | | | 3,0 |
| Net-DTB (10% in Butylene Glycol) | 1,00 | 1,00 | 1,00 | | |
| Sodium Hydroxide (50% in water) | | | | q.s | q.s |
| Disodium EDTA | | | | 0,1 | 0,2 |

| | | | | | |
|---|---|---|---|---|---|
| ²⁹Covacryl E 14, Sensient ³⁰Avalure UR-450^{®} Lubrizol ³¹Pemulen TR-2, Lubrizol | | | | | |

### 3.2 Foundation

| | 1 | 1 |
|---|---|---|
| Deionized Water | ad. 100 | ad. 100 |
| Cellulose Gum | 0,30 | 0,30 |
| Magnesium Aluminum Silicate | 0,35 | 0,35 |
| Lecithin | 0,40 | 0,40 |
| Triethanolamine 99% | 1,25 | 1,25 |
| Butylene Glycol | 6,00 | 6,00 |
| Titanium Dioxide (Water Dispersible) | 8,00 | 8,00 |
| Red Iron Oxide | 0,40 | 0,40 |
| Yellow Iron Oxide | 0,80 | 0,80 |
| Black Iron Oxide | 0,10 | 0,10 |
| Colloidal Kaolin | 2,00 | 2,00 |
| Isoeicosane | 10,00 | 10,00 |
| Isostearic Acid | 1,00 | 1,00 |
| Stearic Acid | 2,50 | 2,50 |
| Glyceryl Stearate | 1,50 | 1,50 |
| Tridecyl Trimellitate | 1,00 | 1,00 |
| Glyceryl Stearate SE | 1,00 | 1,00 |
| Acrylates Copolymer³² | | 5,0 |
| Erfindungsgemäßer Polyurethanharnstoff (bezogen auf Feststoff) | 12,0 | 5,0 |
| Wirkstoffe | q.s. | q.s. |
| Parfüm | q.s. | q.s. |
| Konservierungsmittel | q.s. | q.s. |
| Aqua | Ad.100 | Ad.100 |
| | 1 | 1 |
| Deionized Water | ad.100 | ad.100 |
| Cellulose Gum | 0,30 | 0,30 |
| Magnesium Aluminum Silicate | 0,35 | 0,35 |
| Lecithin | 0,40 | 0,40 |
| Triethanolamine 99% | 1,25 | 1,25 |
| Butylene Glycol | 6,00 | 6,00 |
| Titanium Dioxide (Water Dispersible) | 8,00 | 8,00 |
| Red Iron Oxide | 0,40 | 0,40 |
| Yellow Iron Oxide | 0,80 | 0,80 |
| Black Iron Oxide | 0,10 | 0,10 |
| Colloidal Kaolin | 2,00 | 2,00 |
| Isoeicosane | 10,00 | 10,00 |
| Isostearic Acid | 1,00 | 1,00 |
| Stearic Acid | 2,50 | 2,50 |
| Glyceryl Stearate | 1,50 | 1,50 |

| | | |
|---|---|---|
| ³² Covacrl E 14, Sensient | | |

### 3.3 Eyeliner

| | 1 | 2 |
|---|---|---|
| Oleyl Alcohol | 0,5 | 0,5 |
| Propylene Glycol | 7,5 | 7,5 |
| Xanthan Gum | 0,1 | 0,1 |
| Silica | 0,1 | 0,1 |
| Acrylates Copolymer³³ | 7,0 | |
| Erfindungsgemäßer Polyurethanharnstoff (bezogen auf Feststoff) | 3,0 | 20,0 |
| Wirkstoffe | q.s. | q.s. |
| Farbstoffe | q.s. | q.s. |
| Parfüm | q.s. | q.s. |
| Konservierungsmittel | q.s. | q.s. |
| Aqua | Ad.100 | Ad.100 |

| | | |
|---|---|---|
| ³³ Covacryl E 14, Sensient | | |

### 3.4 Bräunungsmittel

| | 1 |
|---|---|
| Dihydroxyaceton | 3,0 |
| Glycerin | 8,0 |
| Cetyl Alcohol | 0,5 |
| Silica | 3,0 |
| Methylglucose Sesquistearate | 2,0 |
| PEG-100 stearate | 1,0 |
| Cyclomethicone | 4,0 |
| Erfmdungsgemäßer Polyurethanharnstoff (bezogen auf Feststoff) | 10,0 |
| Octyldodecanol | 3,0 |
| Dicaprylylcarbonate | 2,0 |
| EDTA | 1,0 |
| Xanthan Gum | 0,3 |
| Sodium Citrate | 0,4 |
| Citric acid | 0,3 |
| Vitamin E- Acetat | 0,5 |
| Wirkstoffe | q.s. |
| Farbstoffe | q.s. |
| Parfüm | q.s. |
| Konservierungsmittel | q.s. |
| Aqua | Ad.100 |

### 3.5 Getönte Tagescreme

| | |
|---|---|
| Rohstoffe | 1 |
| Glyceryl Stearate Citrate | 3,5 |
| Octyldodecanol | 3,0 |
| Cyclomethicone | 3,0 |
| Cetearyl Alcohol | 1,5 |
| Squalane | 2,0 |
| Shea butter | 5,0 |
| Carbomer | 0,5 |
| Glycerin | 10,0 |
| 4-Methylbenzyliden Campher | 5,0 |
| Octyl Methoxycinnamat | 2,5 |
| Octocrylene | 6,0 |
| Butyl Methoxydibenzoylmethane | 2,5 |
| Erfindungsgemäßer Polyurethanharnstoff (bezogen auf Feststoff) | 5,0 |
| EDTA | 1,0 |
| Wirkstoffe | q. s. |
| Farbstoffe | q. s. |
| Parfüm | q. s. |
| Konservierungsmittel | q. s. |
| Aqua | Ad.100 |

### 3.6 Lippenstift

| | 1 |
|---|---|
| Rizinus oil | 3,0 |
| Caprylic/Capric Triglycerides | 3,0 |
| Octyldodecanol | 5,0 |
| Hydrogenated polyisobutene | 3,0 |
| Jojaba oil | 1,0 |
| Lanolin oil | 1,0 |
| PEG 45 / Dodecyl Glycol copolymer | 2,0 |
| Polyglyceryl-3 Diisostearat | 2,4 |
| Cetyl palmitate | 1,0 |
| C20-40 Alkyl Stearate | 8,0 |
| Carnauba wax | 2,0 |
| Microcristalline wax | 8,0 |
| Glycerin | 10,0 |
| Erfindungsgemäßer Polyurethanharnstoff (bezogen auf Feststoff) | 15,0 |
| Wirkstoffe | q.s. |
| Farbstoffe | q.s. |
| Parfüm | q.s. |
| Konservierungsmittel | q.s. |
| Aqua | Ad.100 |

### 3.7 Gegossenes Blush

| | 1 |
|---|---|
| Caprylic/capric triglyceride | Ad 100 |
| Microcrystalline wax | 3,5 |
| Cera alba | 4,0 |
| C20-40 alkyl stearate | 6,0 |
| Cetyl palmitate | 2,5 |
| Isononyl isononanoate | 6,0 |
| Lauroyl Lysin | 1,0 |
| Nylon | 4,0 |
| PVP/Eicosene Copolymer | 2,0 |
| Tocopheryl acetate | 1,0 |
| Erfindungsgemäßer Po-lyurethanharnstoff (bezogen auf Feststoff) | 5,0 |
| Wirkstoffe | q.s |
| Farbstoffe | q.s |
| Parfüm | q.s |
| Konservierungsmittel | q.s |

### 4. Deodorantien and Antitranspirantien

### 4.1 Deospray

| | 1 | 2 | 3 |
|---|---|---|---|
| Deoparfümöl | 2,5 | | 4,0 |
| Aluminium Chlorohydrate (50% in Wasser) | | 30 | |
| Disteardimonium Hectorite | | 2,0 | |
| Stearalkonium Bentonite | | | 3,0 |
| Propylencarbonat | | 0,8 | |
| Parfüm | q. s. | q. s. | q. s. |
| Dimethicone | | 2 | |
| Cyclomethicone | | | 5 |
| Propylene Glycol | | | 10 |
| Wasser | Ad 100 | | 1,0 |
| Lösungsvermittler | q. s. | q.s | q.s |
| Ethanol | 65,0 | 5 | Add 100 |
| Treibgas | q. s. | Add 100 | q. s. |
| Erfindungsgemäßer Polyurethanharnstoff (bezogen auf Feststoff) | 10,0 | 7,0 | 5,0 |

### 4.2 Deo/AT Pumpspray

| | 1 | 2 |
|---|---|---|
| Deoparfümöl | 2,5 | |
| Aluminium Chlorohydrate (50% in Wasser) | | 25,0 |
| Bisadolol | | 0,01 |
| Ceteareth-20 | | 2,0 |
| Coco Caprylate/Caprate | | 4,50 |
| Dicaprylylether | | 5,0 |
| Glycerin | | 3,0 |
| Glycerylstearate, Ceteareth-20, Cetearylalcohol, Cetylpalmitate, Ceteareth-12 | | 3,8 |
| Parfüm | q.s. | q.s. |
| Lösungsvermittler | q.s | q.s |
| Ethanol | 60,0 | |
| Water | Ad 100 | Ad 100 |
| PEG-100 | 2,0 | |
| Erfindungsgemäßer Polyurethanharnstoff (bezogen auf Feststoff) | 5,0 | 10,0 |

### 4.3 Antitranspirant-Rollergel

| Rohstoffe | 1 | |
|---|---|---|
| Aluminium Chlorohydrate (50% in Wasser) | 20,0 | 30,0 |
| Hydroxyethylcellulose | 0,5 | |
| Ethanol | 40 | |
| Water | Ad. 100 | Ad. 100 |
| Bisabolol | | 0,01 |
| Kamillenextrakt | | 0,1 |
| PPG-15 Stearylether | | 3,0 |
| Avocadoöl | | 0,10 |
| Steareth-2 | | 2,50 |
| Steareth-21 | | 1,50 |
| EDTA | | 1,5 |
| Farbstoffe | q.s | q.s |
| Parfüm | q.s | q.s |
| Lösungsvermittel | q.s | q.s |
| Erfindungsgemäßer Polyurethanharnstoff (bezogen auf Feststoff) | 7,5 | 15,0 |

### 4.4 Antitranspirant-Stift

| | 1 |
|---|---|
| Al-Zr-Tetrachlorohydrex Glycerin (35 wt.% aq. Lsg) | 40,0 |
| Stearyl alcohol | 20,0 |
| Glycerylstearate + PEG-100 stearate | 1,0 |
| Talc | 1,5 |
| PEG-20 | 5,0 |
| Aerosil | 1,5 |
| Cyclopentasiloxane | Ad. 100 |
| Farbstoffe | q.s |
| Parfüm | q.s |
| Lösungsvermittel | q.s |
| Erfindungsgemäßer Polyurethanharnstoff (bezogen auf Feststoff) | 7,5 |

### 4.5 Deo/AT Creme

| | 1 | 2 |
|---|---|---|
| Glyceryl sterarate | 4,0 | 3,0 |
| PEG-2000 stearate | 4,5 | |
| PEG-40 stearate | | 3,5 |
| Cetyl alcohol | 5,0 | 3,0 |
| Cyclopentasiloxane | 6,0 | |
| Mineral oil | 4,5 | 5,0 |
| Avocado oil | 0,1 | 0,10 |
| Kamillenextrakt | | 0,10 |
| C12-15 Alkyl Benzoate | | 0,50 |
| C13-16 Isoparaffin | | 5,0 |
| Bisabolol | | 0,01 |
| Water | Ad.100 | Ad.100 |
| Trisodium EDTA | | 1,50 |
| Konservierungsmittel | q. s. | q. s. |
| Farbstoffe | q.s | q.s |
| Deoparfümöl | q.s | |
| Parfüm | q. s. | q. s. |
| Aluminium Chlorohydrate (50% in Wasser) | | 30,0 |
| Lösungsvermittel | q.s | q.s |
| Erfindungsgemäßer Polyurethanharnstoff (bezogen auf Feststoff) | 15 | 8,0 |

### 4.6 Roller Deo O/W Emulsion

| | 1 |
|---|---|
| Glyceryl laurate | 1,0 |
| Glyceryl stearate | 4,0 |
| Cetyl alcohol | 3,0 |
| Octyldodecanol | 5,0 |
| Ethanol | 10,0 |
| Glycerin | 5,0 |
| Carbomer³⁴ | 0,6 |
| Water | Ad.100 |
| Konservierungsmittel | q.s. |
| Farbstoffe | q.s |
| Deoparfümöl | q.s |
| Neutralisierungsmittel | q.s |
| Lösungsvermittel | q.s |
| Erfindungsgemäßer Polyurethanharnstoff (bezogen auf Feststoff) | 10,0 |

| | |
|---|---|
| ³⁴ Carbopol 980, Lubrizol | |

### 4.7 Mikroemulsion

| | 1 |
|---|---|
| Glyceryl stearate | 2,0 |
| Isosteareth-20 | 4,0 |
| Octyldodecanol | 2,0 |
| Dicaprylyl carbonate | 2,0 |
| Glycerin | 3,0 |
| ACH (50 wt.% Aq. Lsg.) | 20,0 |
| Avocado oil | 0,10 |
| Water | Ad.100 |
| Konservierungsmittel | q.s. |
| Farbstoffe | q.s |
| Deoparfümöl | q.s |
| Neutralisierungsmittel | q.s |
| Lösungsvermittel | q.s |
| Erfindungsgemäßer Polyurethanharnstoff (bezogen auf Feststoff) | 12,0 |

### Shape Memory Effekt (Formgedächtniseffekt.)

### Anwendungstechnisches/e Beispiel/Vergleichsbeispiele:

Für die Untersuchungen des Formgedächtniseffekt wurden kommerziell erhältliche europäische Mischhaare (Nutzlänge: 18 cm, Kerling, Gewicht: 1,0 g) verwendet. Die Haare wurden vor der Anwendung einer standardisierten Waschprozedur unterzogen. Dazu wurde die 15 Minuten in Wasser eingeweichten Haare mit 0,2 mL Standardshampoo eine Minute shampooniert, gründlich mit warmen Wasser ausgespült, kalt trocken geföhnt und bei 21 ± 1 °C und 50 ± 5 % relativer Feuchtigkeit konditioniert. 0,5 g von einer 20 Gew % aktivPolymerlösung ("aktiv": polymerer Filmbildner) wurde auf die Strähne appliziert. Von den so vorbereiteten Haaren wurden Strähnen auf 16 mm Wickler gewickelt und dann bei 21 ± 1 °C und 50 ± 5 % relativer Feuchtigkeit für mindestens 18 Stunden konditioniert. Dann wurden die Haare abgewickelt (= permanente Form). Die Haare wurden zu einer temporären Form mittels eines Gewichts gebracht, das an die Spitze der Haare während 3 Stunden bei Raumtemperatur ausgehängt wurde. Die Haare wurden zu ihrer permanenten Form mittels Wärme (Haartrockner) reaktiviert. Jeder Versuch wurde mit fünf Strähnen durchgeführt.

Der Formgedächtniseffekt wurde durch den Unterschied L_{recov} - Lₚₑᵣₘ quantifiziert, wobei der l_{recov} die Länge der Haarsträhne nach Trocknen und lₚₑᵣₘ ursprüngliche Länge der gelockten Haarsträhne (permanente Form) sind. Je kleiner der Unterschied ist, desto stärker der Formgedächtniseffekt ist.

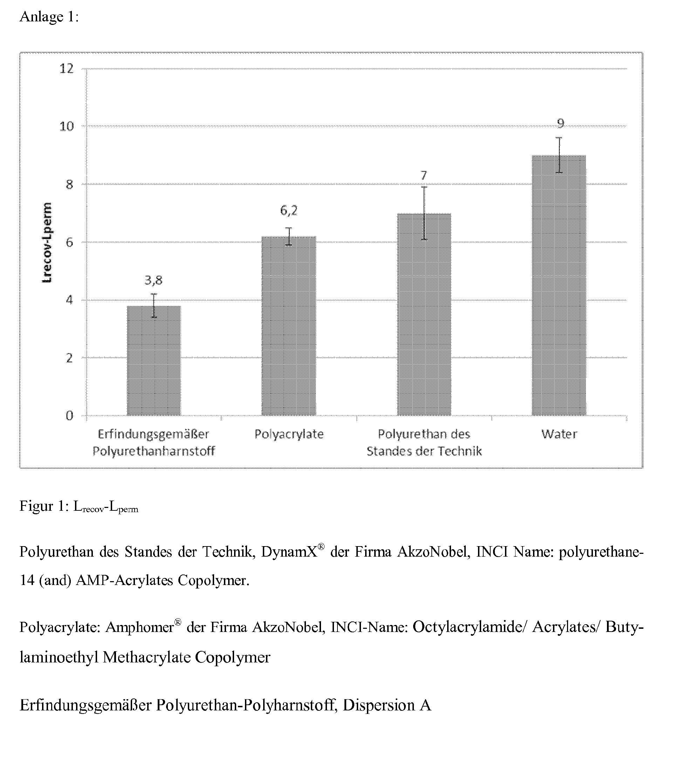

Die Ergebnisse der Messung sind in der Figur 1 (Anlage 1) grafisch dargestellt. Hieraus geht deutlich hervor, dass mit dem erfindungsgemäß verwendeten Polyurethanharnstoff einen besseren Formgedächtniseffekt gegenüber den Polymeren des Standes der Technik erzielt wird.

## Patentansprüche

1. Verwendung mindestens eines Polyurethanharnstoffs zur Herstellung von hautkosmetischen Zusammensetzungen, sowie von haarkosmetischen Zusammensetzungen, wobei letztere ausgewählt sind aus der Gruppe bestehend aus Egalisierungsmittel für Dauerwellen, Curl Relaxer, Styling Wrap Lotion, Haarverformungsmittel, Haarfärbemittel, Haarkuren, Conditioner und Shampoo, **dadurch gekennzeichnet, dass** der Polyurethanharnstoff erhältlich ist durch Umsetzung
a) einer einzigen Polyisocyanat-Komponente,
b) wenigstens einer polymeren Polyolkomponente,
c) wenigstens einer hydrophilierenden Komponente und
d) einer einzigen aminofunktionellen Kettenverlängerer-Komponente,
wobei die Polyisocyanat-Komponente a) mehr als 75 mol% Isophorondiisocyanat und die aminofunktionelle Kettenverlängerer-Komponente c) mehr als 75 mol % Isophorondiamin umfasst.

2. Verwendung von kosmetischen Zusammensetzungen, enthaltend mindestens einen Polyurethanhaarstoff, der erhältlich ist durch Umsetzung
a) einer einzigen Polyisocyanat-Komponente,
b) wenigstens einer polymeren Polyolkomponente,
c) wenigstens einer hydrophilierenden Komponente und
d) einer einzigen aminofunktionellen Kettenverlängerer-Komponente,
wobei die Polyisocyanat-Komponente a) mehr als 75 mol% Isophorondiisocyanat und die aminofunktionelle Kettenverlängerer-Komponente c) mehr als 75 mol % Isophorondiamin umfasst,
in der Haut-, bzw. Haarkosmetik, wobei letztere das Stylen durch Dauerwellen, Curl Relaxer, Styling Wrap Lotions, Haarverformungsmittel, sowie das Färben, Reinigen und Pflegen durch Haarfärbemittel, Haarkuren, Conditioner und Shampoo umfasst.

3. Verwendung von kosmetischen Zusammensetzungen in der Hautkosmetik gemäß Anspruch 2 als Hautpflegeprodukt, Sonnenschutzmittel, After-sun-Präparate, Selbstbräunungsmittel, dekorative Kosmetik, Wasch-, Dusch- und Badepräparate, Gesichtwasser, Gesichtmasken, Insekten-Repellent-Präparate, Fußpflegemittel, Rasiermittel, Haarentfernungsmittel, Intimpflegemittel, Babypflegemittel, Deodorantien und Antitranspirantien.

4. Verwendung von kosmetischen Zusammensetzungen als dekorative Kosmetik gemäß Anspruch 3, insbesondere als Mascara.

5. Verwendung einer Zusammensetzung enthaltend mindestens einen Polyurethanharnstoff, der erhältlich ist durch Umsetzung
a) einer einzigen Polyisocyanat-Komponente,
b) wenigstens einer polymeren Polyolkomponente,
c) wenigstens einer hydrophilierenden Komponente und
d) einer einzigen aminofunktionellen Kettenverlängerer-Komponente,
wobei die Polyisocyanat-Komponente a) mehr als 75 mol% Isophorondiisocyanat und die aminofunktionelle Kettenverlängerer-Komponente c) mehr als 75 mol % Isophorondiamin umfasst,
zur Erzielung eines Shape-Memory-Effekts auf Körpergewebe.

6. Verwendung gemäß einem der Ansprüche 1 bis 5, **dadurch gekennzeichnet, dass** die Polyisocyanat-Komponente a) ≥ 80 mol%, bevorzugt ≥ 85 mol%, weiter bevorzugt 95 mol% und besonders bevorzugt 100 mol% Isophorondiisocyanat umfasst.

7. Verwendung gemäß einem der Ansprüche 1 bis 6, **dadurch gekennzeichnet, dass** die polymere Polyolkomponente b) zahlenmittlere Molekulargewichte von ≥ 400 und ≤ 8000 g/mol und /oder eine OH-Funktionalitäten von 1,5 bis 6 aufweist.

8. Verwendung gemäß einem der Ansprüche 1 bis 7, **dadurch gekennzeichnet, dass** die polymere Polyolkomponente b) einen Polyester, bevorzugt einen Polyester basierend auf Adipinsäure umfasst oder daraus besteht.

9. Verwendung gemäß einem der Ansprüche 1 bis 8, **dadurch gekennzeichnet, dass** die hydrophilierende Komponente c) eine anionisch hydrophilierende Komponente und bevorzugt ein Sulfonat ist.

10. Verwendung gemäß Anspruch 9, **dadurch gekennzeichnet, dass** die aminofunktionelle Kettenverlängerer-Komponente d) ≥85 mol%, bevorzugt ≥ 95 mol% und besonders bevorzugt 100 mol% Isophorondiamin umfasst.

11. Verwendung gemäß einem der Ansprüche 2 bis 10, wobei die Zusammensetzungen als weitere Komponenten Öle, Fette , Wachse, Verdickungsmittel, Emulgatoren, UV-Filter, Farbstoffe, Konditionierungsmittel, Tenside, Filmbildner, Lösungsmittel, Treibgase, wirkstoffe und oder sonstige Hilfs-und/oder Zusatzstoffe enthalten.

12. Verfahren zur Reinigung, zur Pflege und/oder zum Schutz der Haut, bzw. zum Erzielen eines stylenden Effekts der Haare mittels Dauerwellen, eines Effekts zum Glätten der Haare (Curl Relaxer), eines Effekts zum Einbringen von Locken in die Haare (Styling Wrap Lotion), eines haarverformenden Effekts, eines färbenden, reinigenden, oder pflegenden Effekts der Haare durch Auftragen einer Zusammensetzung enthaltend mindestens einen Polyurethanharnstoff, der erhältlich ist durch Umsetzung
a) einer einzigen Polyisocyanat-Komponente,
b) wenigstens einer polymeren Polyolkomponente,
c) wenigstens einer hydrophilierenden Komponente und
d) einer einzigen aminofunktionellen Kettenverlängerer-Komponente,
wobei die Polyisocyanat-Komponente a) mehr als 75 mol% Isophorondiisocyanat und die aminofunktionelle Kettenverlängerer-Komponente c) mehr als 75 mol % Isophorondiamin umfasst,
auf Körpergewebe.

13. Verfahren gemäß Anspruch 12, wobei die Zusammensetzung zumindest teilweise auf dem Körpergewebe verbleibt.

14. Verfahren zum Fixieren des Wimpernschwungs durch Auftragen einer Zusammensetzung, enthaltend mindestens einen Polyurethanharnstoff, der erhältlich ist durch Umsetzung
a) einer einzigen Polyisocyanat-Komponente,
b) wenigstens einer polymeren Polyolkomponente,
c) wenigstens einer hydrophilierenden Komponente und
d) einer einzigen aminofunktionellen Kettenverlängerer-Komponente,
wobei die Polyisocyanat-Komponente a) mehr als 75 mol% Isophorondiisocyanat und die aminofunktionelle Kettenverlängerer-Komponente c) mehr als 75 mol % Isophorondiamin umfasst,
auf die Augenwimpern.

15. Verfahren zur Erzielung eines Shape-Memory-Effekts auf Körpergewebe, bei dem kosmetische Zusammensetzungen, enthaltend mindestens einen Polyurethanharnstoff, der erhältlich ist durch Umsetzung
a) einer einzigen Polyisocyanat-Komponente,
b) wenigstens einer polymeren Polyolkomponente,
c) wenigstens einer hydrophilierenden Komponente und
d) einer einzigen aminofunktionellen Kettenverlängerer-Komponente,
wobei die Polyisocyanat-Komponente a) mehr als 75 mol% Isophorondiisocyanat und die aminofunktionelle Kettenverlängerer-Komponente c) mehr als 75 mol % Isophorondiamin umfasst,
auf Haut oder Haare aufgetragen werden.

16. Verfahren gemäß einem der Ansprüche 12 bis 15, wobei die Zusammensetzungen als weitere Komponenten Öle, Fette , Wachse, Verdickungsmittel, Emulgatoren, UV-Filter, Farbstoffe, Konditionierungsmittel, Tenside, Filmbildner, Lösungsmittel, Treibgase, wirkstoffe und oder sonstige Hilfs-und/oder Zusatzstoffe enthalten.
